# EUROPEAN PATENT SPECIFICATION

(11) **EP 3 589 956 B1**
(45) Date of publication and mention of the grant of the patent: **23.04.2025**
(21) Application number: 18712334.4
(22) Date of filing: 02.03.2018
(51) Int. Cl.: G01N 33/569, C12N 15/864, G01N 33/48

(54) **METHODS FOR DETERMINING POTENCY OF ADENO-ASSOCIATED VIRUS PREPARATIONS**
VERFAHREN ZUR BESTIMMUNG DER WIRKSTÄRKE VON ADENO-ASSOZIIERTEN VIRUSPRÄPARATEN
PROCÉDÉS DE DÉTERMINATION DE L'ACTIVITÉ THÉRAPEUTIQUE DE PRÉPARATIONS DE VIRUS ADÉNO-ASSOCIÉS

(30) Priority: 03.03.2017 US 201762467045 P
(43) Date of publication of application: 08.01.2020
(73) Proprietor: Takeda Pharmaceutical Company Limited, Osaka-shi, Osaka (JP)
(72) Inventor: BENDÍK, Marián, 84101 Bratislava (SK); NECINA, Roman, 1210 Vienna (AT); BOEHM, Ernst, 1210 Vienna (AT); GRANINGER, Michael, 1150 Vienna (AT); FIEDLER, Christian, 1100 Vienna (AT)
(74) Representative: Hoffmann Eitle
(86) International application number: PCT/US2018/020676
(87) International publication number: WO 2018/160975

(56) References cited:
- WO-A1-2013/063379
- GRIMM D ET AL: "TITRATION OF AAV-2 PARTICLES VIA A NOVEL CAPSID ELISA: PACKAGING OF GENOMES CAN LIMIT PRODUCTION OF RECOMBINANT AAV-2", GENE THERAPY, NATURE PUBLISHING GROUP, LONDON, GB, vol. 6, no. 7, 1 July 1999 (1999-07-01), pages 1322 - 1330, XP000892113, ISSN: 0969-7128, DOI: 10.1038/SJ.GT.3300946
- STEPHANIE KRONENBERG ET AL: "Electron cryo-microscopy and image reconstruction of adeno-associated virus type 2 empty capsids", EMBO REPORTS, vol. 2, no. 11, 1 November 2001 (2001-11-01), GB, pages 997 - 1002, XP055470096, ISSN: 1469-221X, DOI: 10.1093/embo-reports/kve234
- BRITTA GERLACH ET AL: "Conformational Changes in Adeno-Associated Virus Type 1 Induced by Genome Packaging", JOURNAL OF MOLECULAR BIOLOGY, ACADEMIC PRESS, UNITED KINGDOM, vol. 409, no. 3, 28 March 2011 (2011-03-28), pages 427 - 438, XP028296581, ISSN: 0022-2836, [retrieved on 20110402], DOI: 10.1016/J.JMB.2011.03.062
- BÖTTCHER B. ET AL: "Difference imaging reveals ordered regions of RNA in turnip yellow mosaic virus", STRUCTURE, vol. 4, no. 4, 1 April 1996 (1996-04-01), AMSTERDAM, NL, pages 387 - 394, XP055804663, ISSN: 0969-2126, DOI: 10.1016/S0969-2126(96)00044-5
- DUBOCHET J ET AL: "Cryo-electron microscopy of vitrified specimens", QUARTERLY REVIEWS OF BIOPHYSICS, CAMBRIDGE UNIVERSITY PRESS, GB, vol. 21, no. 2, 1 January 1988 (1988-01-01), pages 129 - 228, XP008091885, ISSN: 0033-5835, DOI: 10.1017/S0033583500004297
- R. M. KOTIN: "Large-scale recombinant adeno-associated virus production", HUMAN MOLECULAR GENETICS, vol. 20, no. R1, 15 April 2011 (2011-04-15), pages R2 - R6, XP055016870, ISSN: 0964-6906, DOI: 10.1093/hmg/ddr141
- SMITA BHATIA: ""Combined Crystallographic and Cryo-Electron Microscopic Analysis of Adeno-Associated Virus Type 2"", 15 July 2003 (2003-07-15), XP009505078, Retrieved from the Internet <URL:https://www.google.com/url?sa=t&rct=j&q=&esrc=s&source=web&cd=4&cad=rja&uact=8&ved=0ahUKEwjFj7vQ4tLaAhURMuwKHSQFBFkQFgg_MAM&url=http%3A%2F%2Ffsu.digital.flvc.org%2Fislandora%2Fobject%2Ffsu%3A175930%2Fdatastream%2FPDF%2Fdownload%2Fcitation.pdf&usg=AOvVaw2WXzO9Rf6TpTa3eyitxcMH> [retrieved on 20180503]
- STEPHANIE KRONENBERG ET AL: "Electron cryo-microscopy and image reconstruction of adeno-associated virus type 2 empty capsids", EMBO REPORTS, vol. 2, no. 11, 1 November 2001 (2001-11-01), GB, pages 997 - 1002, XP055470096, ISSN: 1469-221X, DOI: 10.1093/embo-reports/kve234

## Description

### RELATED APPLICATIONS

This application claims priority to U.S. Provisional Patent Application No. 62/467,045 filed March 3, 2017.

### BACKGROUND

Adeno-associated virus (AAV) is a small, non-enveloped virus that packages a linear single-stranded DNA genome. AAV belongs to the family *Parvoviridae* and the genus *Dependovirus,* since productive infection by AAV occurs only in the presence of a helper virus, such as, for example, adenovirus or herpes virus. Even in the absence of a helper virus, AAV (serotype 2) can achieve latency by integrating into chromosome 19q13.4 of a host human genome. It is the only mammalian DNA virus known to be capable of site-specific integration (Daya and Berns, Clinical Microbiology Reviews, pages 583-593 (2008)).

For AAV to be safely used in the clinic, AAV has been genetically modified at several locations within its genome. For example, the *Rep* gene, which is required for viral replication, and the element required for site-specific integration have been eliminated from the AAV genome in many viral vectors. This recombinant AAV (rAAV) exists in an extrachromosomal state and has very low integration efficiency into the genomic DNA. The possibility of rAAV inducing random mutagenesis in a host cell is thus reduced, if not eliminated altogether. Because of these properties and the lack of pathogenicity, rAAV has shown great promise as a gene therapy vector in multiple aspects of pre-clinical and clinical applications. New serotypes and self-complementary vectors are being tested in the clinic. Alongside these ongoing vector developments, continued effort has focused on scalable manufacturing processes that can efficiently generate high titer quantities of rAAV vectors with high purity and potency.

Although the effort to design efficient, large-scale methods to purify an AAV product suitable for human administration has advanced, there still remains a need for analytical methods to measure quality attributes of gene therapy vector preparations based on recombinant rAAV, to allow product release and accurate clinical dosing. For example, current methods of generating AAV in cell culture result in the formation of "empty" capsids, which lack the vector genome and have been shown to lead to T-cell-mediated immune responses. Moreover, empty capsids are unable to provide a therapeutic benefit associated with transgene production, and there is a potential for increasing innate or adaptive immune responses to the vector (e.g., T-cell mediated), which then renders empty capsids a concern in gene therapy contexts. Wright, Molecular Therapy 22: 1-2 (2014). WO2013/063379 A1 relates to the development of a scalable manufacturing process for AAV vectors.

Classically, the use of quantitative PCR (qPCR) is the most widely accepted and preferred to method for determining the dose of AAV preparations because it provides a measurement of the vector genomes (e.g., vector genome (vg)/kg subject). *See* Halbert CL et al., J Virol 1997;71:5932-5941; Samulski RJ et al., J Virol 1989;63:3822-3828; Clark KR et al., Hum Gene Ther 1999;10:1031-1039; and Wright JF, Hum Gene Ther 2011;22:519-521. Use of qPCR is limited, however, by the fact that only total DNA is measured, which can lead to significant issues. For example, the readout of qPCR can be impacted by issues such as, the use of inefficient primers, issues with calibration standards, or DNA tertiary structures (e.g., hairpin structures, circular, or supercoiled). Thus, the use of qPCR can lead to inaccurate determination of the dose and ultimately potency of the final AAV preparation due to the inclusion of capsids that contain more than one copy of DNA and/or fractured capsids in the dose calculation. This is because the potency of an AAV preparation consist of two parts: 1) the ability of AAV capsid to transduce cells and 2) the ability of DNA in AAV to produce desired product. Inoperable capsids, which would not be accounted for with qPCR, do not effectively transduce the cells. Moreover, qPCR is also limited due to its inherent variability (i.e., high variability up to 0.5 log steps) which can lead to an inaccurate determination of dose of the final AAV preparation. The inherent variability can also be compounded by the inter-assay variability seen with qPCR due to sample preparation, which involves several steps, including, capsid protein digestion, DNA extraction, and DNA purification. If the dose and/or potency is not accurately determined, patients may receive the wrong dose and/or widely varying doses from treatment to treatment. Thus, an accurate tool to measure the dose and ultimately the potency of AAV preparations, and to monitor gene therapy vector quality and potency is therefore needed.

### SUMMARY

The invention is defined in the appended claims.

Provided herein are methods of measuring the quantity attributes of gene therapy vector preparations. In certain embodiments, the gene therapy vector preparations are adeno-associated virus (AAV) preparations. The methods disclosed herein can be used at any point of purification. In certain embodiments, the methods can be used prior to preparing or packaging the AAV capsids for use. The methods disclosed herein can be used to improve the determination of the concentration, dose, and/or potency of an AAV preparation.

A feature of AAV vector generation in cell culture is the formation of an excess of "empty" capsids, which lack the vector genome. In certain embodiments, the amount of empty capsids is assessed in order to accurately determine the concentration, dose, and/or potency of an AAV preparation. Empty capsids can also be considered as product related impurities, which should be reduced during purification. In certain embodiments, it is important to determine the total amount of empty capsids to reduce an innate and/or adaptive immune response against the capsid antigen.

Classically, the concentration, dose, and/or potency of an AAV preparation relies on a quantitative determination by quantitative PCR (qPCR). Surprisingly, the inventors found that an AAV-specific enzyme-linked immunosorbent assay (ELISA) alone can be relied upon for the quantitative determination of the AAV preparation and its use can improve the measurement of the concentration and/or dose an AAV preparation and ultimately the potency. The methods of the present disclosure are advantageous over those known in the art because antigen determination with ELISA provides highly reliable results, surprisingly more accurate than that of qPCR. In certain embodiments, the method does not include a step of measuring concentration, dose, and/or potency via qPCR. In certain embodiments, the concentration, dose, and/or potency can be determined by the concentration of AAV capsids in an AAV preparation. In certain embodiments, the methods disclosed herein comprising ELISA result in an AAV preparation with at least about 10% less variability of the concentration, dose, and/or potency as compared to a method using qPCR. In certain embodiments, the methods disclosed herein comprising ELISA result in an AAV preparation with at least about 20% less variability of the concentration, dose, and/or potency as compared to a method using qPCR. In certain embodiments, the methods disclosed herein comprising ELISA result in an AAV preparation with about 10% to about 80% less variability of the concentration, dose, and/or potency as compared to a method using qPCR. In certain embodiments, the methods disclosed herein comprising ELISA result in an AAV preparation with about 15% to about 50% less variability of the concentration, dose, and/or potency as compared to a method using qPCR. In certain embodiments, the methods disclosed herein comprising ELISA result in an AAV preparation with about 20% to about 33% less variability of the concentration, dose, and/or potency as compared to a method using qPCR.

In certain aspects, methods of the invention entail quantifying the dose and/or potency of an AAV preparation. In certain embodiments, the method comprises testing an AAV preparation via an AAV-specific ELISA assay to quantify the total number of AAV capsids in the AAV preparation. The number of capsids can then be used to determine the dose to administer a subject. The dose can be used to determine the potency (i.e., the dose or concentration required to produce a certain effect (e.g., EC₅₀)). In certain embodiments, the method does not include a step of measuring the dose and/or potency via qPCR.

In certain aspects, methods of the invention entail measuring the concentration of AAV capsids in an AAV fraction or preparation. In certain embodiments, the method comprises testing an AAV fraction or preparation via an AAV-specific ELISA assay to quantify the total number of AAV capsids in the AAV fraction or preparation. In certain embodiments, the method does not include a step of measuring the concentration via qPCR.

In certain aspects, methods of the invention entail measuring the concentration of full AAV capsids in an AAV fraction or preparation. In all embodiments of the invention, the method comprises testing an AAV fraction or preparation via an AAV-specific ELISA assay to quantify the total number of AAV capsids in the AAV fraction or preparation and evaluating the percentage or ratio of full versus empty AAV capsids in the AAV preparation using cryogenic transmission electron microscopy (CryoTEM)). In certain embodiments, the method does not include a step of measuring the concentration via qPCR.

In certain embodiments, empty capsids are removed from the AAV fraction or preparation resulting in an AAV fraction or preparation with a specific concentration of full capsids and/or a specific ratio of full:empty capsids. In certain embodiments, the concentration or ratio is constant between batches such that an AAV-specific ELISA assay is sufficient to determine the concentration, dose, and/or potency of the AAV fraction or preparation. In certain embodiments, the method does not include a step of measuring the concentration, dose, and/or potency via qPCR.

In certain aspects, methods of the invention entail quantifying the dose and/or potency of an AAV preparation. The method comprises testing an AAV preparation via an AAV-specific ELISA assay in combination with a method that evaluates or confirms the percentage or ratio of full versus empty (full:empty) AAV capsids in the AAV preparation by CryoTEM. In certain embodiments, the method does not include a step of measuring the dose and/or potency via qPCR.

In certain embodiments, the method comprises measuring the concentration of AAV capsids in an AAV fraction or preparation via an AAV-specific ELISA assay in combination with CryoTEM that evaluates or confirms the percentage or ratio of full:empty AAV capsids in the AAV fraction or preparation. In certain embodiments, the method does not include a step of measuring the concentration via qPCR.

In certain embodiments, the method for quantifying the dose and/or potency of an AAV preparation comprises confirming the biological activity of the AAV preparation. In certain embodiments, the method comprises confirming the biological activity of the AAV preparation using an *in vitro* method. In certain embodiments, the methods of the invention do not require confirmation using a biological assay because the AAV-specific ELISA assay is paired with a CryoTEM method to confirm product quality (i.e., measuring the ratio of full:empty). In certain embodiments, the method does not include a step of measuring dose and/or potency via qPCR.

In certain aspects, methods of the invention entail producing an AAV preparation, comprising: (i) transfecting host cells in cell culture with at least one plasmid comprising a gene of interest;
(ii) collecting supernatant or cell suspension of the cell culture comprising AAV capsids to create an AAV fraction; (iii) quantifying the total number of AAV capsids in the AAV fraction using an AAV-specific ELISA assay; and (iv) preparing an AAV preparation with a desired concentration of AAV capsids based on the total number of AAV capsids determined in step (iii). In certain embodiments, the method comprises concentrating the AAV fraction. In certain embodiments, the method comprises removing at least a portion of empty capsids from the AAV fraction. In certain embodiments, at least a portion of the empty capsids are removed to create an AAV fraction or preparation with a specific concentration of full capsids and/or a specific ratio of full:empty capsids. In all embodiments of the invention, the method comprises evaluating the percentage or ratio of full versus empty (full:empty) AAV capsids in the AAV preparation by CryoTEM. In certain embodiments, the method does not include a step of measuring the concentration, dose, and/or potency via qPCR. In certain embodiments, the method further comprises lyophilizing the AAV fraction or preparation.

In certain embodiments, the ELISA assay can be a sandwich ELISA, direct ELISA, indirect ELISA, or competitive ELISA assay. In certain embodiments, for the methods disclosed herein, the AAV-specific ELISA assay is a sandwich ELISA assay specific for an AAV antigen. In certain embodiments, the AAV antigen is an AAV1, AAV2, AAV3, AAV4, AAV5, AAV6, AAV7, AAV8, AAV9, AAV10, or a chimeric AAV antigen. In certain embodiments, the AAV antigen is an AAV8 antigen. In certain embodiments, the AAV antigen is from a recombinant AAV (rAAV). In certain embodiments, the AAV antigen is from a genetically engineered AAV, or chemically modified AAV, or both.

In certain embodiments, the ELISA comprises an antibody specific for an AAV epitope of the antigen. In certain embodiments, the AAV epitope is a conformational epitope present on assembled AAV capsids. In certain embodiments, the AAV epitope is a linear epitope present on assembled AAV capsids. Examples of such epitopes include, but is not limited to, capsid virion proteins VP1, VP2, and/or VP3. In certain embodiments, the AAVs are genetically engineered to express additional virion proteins on the surface of the capsid, and those engineered proteins can be used/detected in an ELISA assay. In certain embodiments, the AAV are chemically modified to express variants of virion protein, which can be used/detected in an ELISA assay (e.g., VP1', VP2', VP3', etc...). In certain embodiments, the antibodies identify serotype specific capsid virion proteins.

In all embodiments of the invention, the method for evaluating or confirming the percentage or ratio of full:empty AAV capsids in an AAV fraction or preparation is CryoTEM. In certain embodiments, evaluating comprises quantitating the percentage or ratio of full:empty AAV capsids.

In certain embodiments, the CryoTEM step comprises: (i) embedding an AAV fraction or preparation in a substrate in an inert support; (ii) flash-freezing the embedded AAV fraction or preparation; (iii) imaging the embedded AAV fraction or preparation using cryogenic transmission electron microscopy; and (iv) quantitating the percentage of full AAV capsids versus empty AAV capsids in the AAV fraction or preparation. In certain embodiments, the substrate is amorphous, non-crystalline ice. In certain embodiments, the AAV fraction or preparation is free from cellular debris. For example, the vector samples do not contain uncertain particles >2%, does not contain broken or oligomerized particles >2%.

The use of CryoTEM in combination with an AAV-specific ELISA assay is advantageous over those known in the art because CryoTEM provides a direct measurement of full:empty capsids, unlike that of quantitative PCR (qPCR). In certain embodiments, the method does not include a step of measuring concentration or dose via qPCR.

In certain embodiments, for the methods disclosed herein, the ELISA assay is performed prior to the quantification of full:empty AAV capsids. In certain embodiments, the ELISA assay is performed prior to CryoTEM. In certain embodiments, the ELISA assay is performed to dilute the total AAV capsid number to a validated range (e.g., the cp/ml ranges as disclosed herein). In certain embodiments, the ELISA assay is performed after the quantification of full:empty AAV capsids. In certain embodiments, the ELISA assay is performed after CryoTEM. In certain embodiments, the ELISA assay is performed to dilute the total of full AAV capsid number to a validated range. In certain embodiments, the quantification of full:empty is performed only once. For example, the CryoTEM step can be used once to test that the batch has a consistent empty:full ratio as that of the previous batches.

The use of both ELISA and CryoTEM results in a method that can be applied for all AAV serotypes independent from vector DNA. This is advantageous as it allows the assay to be performed across all platforms.

In certain embodiments, for the methods, fractions or preparations disclosed herein, the percentage of full AAV capsids in the AAV fraction or preparation as compared to empty capsids is from about 40% to about 100%. In certain embodiment, the percentage of full AAV capsids is from about 40% to about 95%, about 40% to about 90%, about 40% to about 85%, about 40% to about 80%, about 45% to about 75%, about 50% to about 70%, or about 55% to about 65%. In certain embodiments, at least about 60% of the AAV capsids are full AAV capsids. In certain embodiments, at least about 40%, at least about 50%, at least about 60%, at least about 70%, at least about 80%, at least about 85%, at least about 90%, at least about 91%, at least about 92%, at least about 93%, at least about 94%, at least about 95%, at least about 96%, at least about 97%, at least about 98%, at least about 99%, or at least about 100% of the AAV capsids are full AAV capsids. In certain embodiments, the methods as disclosed herein are used to generate AAV fractions or preparations with consistent amounts of full capsids between batches of AAV fractions or preparations.

In certain embodiments, for the methods and preparations disclosed herein, the concentration of the AAV preparations is between about 1×10¹⁰ cp/ml to about 1×10²⁰ cp/ml. In certain embodiments, for the methods and preparations disclosed herein, the concentration of the AAV preparations is between about 1×10¹¹ cp/ml to about 1×10¹⁹ cp/ml, about 1×10¹² cp/ml to about 1×10¹⁸ cp/ml, about 1×10¹³ cp/ml to about 1×10¹⁷ cp/ml, or about 1×10¹⁴ cp/ml to about 1×10¹⁶ cp/ml. In certain embodiments, for the methods and preparations disclosed herein, the concentration of the AAV preparations is between about 1×10¹² cp/ml to about 1×10¹⁵ cp/ml, about 1×10¹³ cp/ml to about 1×10¹⁵ cp/ml, or about 1×10¹² cp/ml to about 1×10¹³ cp/ml. In certain embodiments, for the methods and preparations disclosed herein, the concentration of the AAV preparations is between about 1×10¹⁴ cp/ml to about 5×10¹⁴ cp/ml, about 2×10¹⁴ cp/ml to about 3×10¹⁴ cp/ml, or about 3.5×10¹⁴ cp/ml to about 5×10¹⁵ cp/ml. In certain embodiments, cp/ml can be total capsids per ml or full capsids per ml. In certain embodiments, the cp/ml is total capsids per ml.

In certain embodiments, for the methods or preparations disclosed herein, the dose of the AAV preparation is between about 1 x 10⁵ cp/kg to about 1 x 10²⁵ cp/kg. In certain embodiments, for the methods, fractions, or preparations disclosed herein, the dose of the AAV fraction or preparation is between about 1 x 10¹⁰ cp/kg to about 1 x 10²⁰ cp/kg. In certain embodiments, for the methods, fractions, or preparations disclosed herein, the dose of the AAV fraction or preparation is between about 1 x 10¹⁰ cp/kg to about 1 x 10¹⁶ cp/kg. In certain embodiments, cp/kg can be total capsids per kg of the subject or full capsids per kg of the subject. In certain embodiments, the cp/kg is total capsids per kg of the subject.

In certain embodiments, for all methods disclosed herein, the AAV is AAV1, AAV2, AAV3, AAV4, AAV5, AAV6, AAV7, AAV8, AAV9, AAV10, or a chimeric AAV vector. In certain embodiments, the AAV is AAV8. In certain embodiments, the AAV is AAV9. In certain embodiments, the AAV is a recombinant AAV (rAAV). In certain embodiments, the AAV is genetic engineered, chemically modified, or both.

### BRIEF DESCRIPTION OF THE DRAWINGS

Figure 1 is a graph comparing the percentage of errors for the measurement of AAV vector number and vector genome content using antigen ELISA or qPCR, respectively. AAV ELISA (QC ("Quality Control")): n=60; AAV ELISA (R&D ("Research and Development")): n=83; ITR-qPCR (R&D): n=64; FIX-qPCR (QC): n=40; FIX-qPCR (R&D): n=135.
Figure 2 is the result from an agarose gel electrophoresis study examining the ability of antigen ELISA and qPCR to estimate the dose of AAV capsids loaded onto the agarose gel (2.0×10¹⁰ capsids/lane).
Figure 3 is a graph verifying use of the antigen ELISA method to determine the dose of the AAV preparation administered in an *in vivo* biopotency assay using a mouse model (n=7).
Figure 4 is a graph comparing batch to batch variation of AAV8 FVIII single stranded DNA using AAV-specific ELISA versus ITR-qPCR. AAV8 : Antigen - Median : 3.03cp/ml ± 0.66cp/ml. ITR-qPCR - Median : 9.11vg/ml ± 3.01vg/ml.
Figure 5A-5B: Figure 5A is a graph demonstrating the ratio of AAV vector genomes (vg) per AAV8 antigen ELISA capsid particles (cp). Figure 5B is a graph demonstrating the high consistency of "full" capsids in the AAV preparation (e.g., see the process as discussed in the publication of International Application No. PCT/US2017/059967).
Figure 6A-6C: Figure 6A is a CryoTEM image of one full AAV8 capsid and one empty AAV8 capsid. Figure 6B is a CryoTEM image of one oligomerized AAV8 capsid (i.e., the AAV capsid is forms a trimeric structure, which could be empty or full). Figure 6C is a CryoTEM image of one broken AAV8 capsid.
Figure 7 is graph of an AAV8 fraction analysis using CryoTEM. CryoTEM images were acquired at 38k magnification and subjected to internal density analysis. The principal component analysis to generate the culsters of each AAV particle's radial density profile revealed clusters of packaging levels: full (left), blue (right), uncertain (middle) (darker marks indicate overlapping data points); Dashed rings: 99% confidence intervals. 5 images analyzed, 1568 particles.
Figure 8 is a graph validating the use of CryoTEM to measure the percentage of full AAV8 vector capsids.
Figure 9 is a graph demonstrating the correlation of vector genomes to capsid particles with percent of full capsids.
Figure 10 is a graph demonstrating the influence of the percentage of full AAV8 capsid particles on the *in vivo* potency in a Factor IX-knockout mouse model. Plasma samples were taken after 14 days and human Factor IX clotting activity was measured.
Figure 11 is a graph demonstrating the influence of the percentage of full AAV8 capsid particles on the *in vitro* biopotency. The biopotency was measured in a cell-based assay, related to cp, and plotted against the percent full particles.
Figure 12 is a graph comparing batch to batch variation of AAV8 FVIII single stranded DNA using AAV-specific ELISA versus ITR-qPCR for batches 17004-17013 (see Table 7).
Figure 13 is a graph demonstrating the high consistency of "full" capsids in the AAV preparation (e.g., see the process as discussed in the publication of International Application No. PCT/US2017/059967) for batches 17004-17013 (see Table 7).

The further attached figures are intended merely to further illuminate the disclosure and do not pose a limitation on the scope of the disclosure unless otherwise claimed.

### DETAILED DESCRIPTION

Provided herein are methods of measuring the qualitative and/or quantity attributes of gene therapy vector preparations. In certain embodiments, the gene therapy vector preparations are AAV preparations, for example, AAV8 or AAV9. All methods disclosed herein can be used on or to generate an AAV preparation.

The use of gene therapy vectors relies on at least two biological activities for its potency: 1) the ability to transfer a DNA sequence to a cell, and 2) the biological effect of the expressed genetic sequence. In order to achieve these activities, the proper concentration or dose of AAV capsids containing the genomic sequence is required. This is because "empty" capsids are unable to provide a therapeutic benefit associated with transgene production. Thus, it is important to accurately measure the concentration or dose of the AAV preparation to ensure the proper and/or consistent number of full AAV vector particles are delivered. This is especially true because for recombinant proteins an appropriate coefficient of variance between lots/dosage amounts is ≤ 10 % to 20 %, with ≤ 10% being more ideal. Proper determination of the dose is important to ascertain the potency of the AAV preparation. This is because potency is determined by graded or quantal dose-response curves. If the dose is inaccurate, the inaccuracy will be transferred to the measured potency. Inaccurate potency could lead to under or over treatment of a patient population.

As used herein, the terms "capsid", "capsid particle", and "particle" are used interchangeably and refer to an AAV particle composed of at least one intact AAV capsid shell.

As used herein, the term "empty" with regard to AAV or AAV capsids refers to those that lack the complete (i.e., full) vector genome. Empty AAV or empty AAV capsids or empty AAV particles are unable to provide a therapeutic benefit. As used herein, the term "full AAV capsids" with regard to AAV or AAV capsids or AAV particles refer to those containing a majority of the complete vector genome. Full AAV capsids can provide a therapeutic benefit to recipient patients. In certain embodiments, "full" can also include "incomplete vector DNA" or "truncated vector DNA". In certain embodiments, complete versus incomplete and/or truncated vector DNA can be differentiate with additional analytic methods. Such methods include, without limitation, DNA sizing by capillary electrophoresis, AUC (analytical ultracentrifugation), % Agarose DNA (native or alkaline), gel, southern blot, dot-blot hybridization, UV spectrophotometry, weak anion exchange chromatography, and mass spectrometry (*See* Resolving Adeno-Associated Viral Particle Diversity with Charge Detection Mass Spectrometry Elizabeth E. Piersonet.al Anal. Chem., 2016, 88 (13), pp 6718-6725).

According to the FDA guidelines published in January 2011(Guidance for Industry: Potency Tests for Cellular and Gene Therapy Products), there are three ways to measure potency such as: 1) *in vivo* biological; 2) *in vitro* non-biological assays; and 3) multiple tests. Biological assays, however, lead to a large amount of variability due to dosing, assay, and readout variability. Non-biological assays have issues as well. For example, quantitative PCR (qPCR) only measures the gene of interest. Thus, the use of qPCR can lead to inaccurate determination of the potency of the final AAV preparation due to the inclusion of inoperable capsids such as those that are oligomerized, fractured, and/or contain more than one copy of DNA in the dose calculation. Inoperable capsids would not effectively transduce the cells. Moreover, qPCR is also limited due to its inherently high intra- and inter-assay variability (i.e., high variability up to 0.5 log steps) which can lead to inaccurate determination of potency. If the concentration, dose and/or potency is not accurately determined, patients may receive widely varying doses from batch to batch and/or treatment to treatment. Over estimating the dose or potency can lead to inadequate treatment of the disease and/or disorder. Under estimating the dose or potency can lead to side effects (e.g., over correction (e.g., blood clots) or an immune response caused by a high number of capsid particles in the body).

In certain embodiments, the invention provides methods of quantifying the concentration, dose, and/or potency of an AAV preparation, comprising quantifying the total number of AAV capsids in an AAV preparation. The method comprises a) quantifying the total number of AAV capsids and b) evaluating and/or confirming the percentage or ratio of full versus empty (full:empty) AAV capsids in the AAV preparation.

The quantifying step comprises determining the total number of AAV capsids via an AAV-specific ELISA. In certain embodiments, the ELISA assay can be a sandwich ELISA, direct ELISA, indirect ELISA, or competitive ELISA assay. In certain embodiments, for the methods disclosed herein, the AAV-specific ELISA assay is a sandwich ELISA assay specific for an AAV antigen.

The method for evaluating the percentage or ratio of full versus empty (full:empty) AAV capsids in an AAV fraction or preparation is CryoTEM. In all embodiments, the methods include both an AAV-specific ELISA and CryoTEM.

The methods disclosed herein accurately measure the potency within this narrow range of variability. In certain embodiments, the disclosed methods result in a coefficient of variation between about 30% to about 1%, about 27% to about 2%, about 25% to about 5%, about 22% to about 7%, about 20% to about 10%, or about 17% to about 12%. In certain embodiments, the disclosed methods result in a coefficient of variation of ≤ about 30%, ≤ about 29%, ≤ about 28%, ≤ about 27%, ≤ about 26%, ≤ about 25%, ≤ about 24%, ≤ about 23%, ≤ about 22%, ≤ about 21%, ≤ about 20%, ≤ about 19%, ≤ about 18%, ≤ about 17%, ≤ about 16%, ≤ about 15%, ≤ about 14%, ≤ about 13%, ≤ about 12%, ≤ about 11%, ≤ about 10%, ≤ about 9%, ≤ about 8%, ≤ about 7%, ≤ about 6%, ≤ about 5%, ≤ about 4%, ≤ about 3%, ≤ about 2%, ≤ 1%, but in all cases ≥ 0.

In certain embodiments, the methods disclosed herein comprising ELISA result in an AAV preparation with about 10% to about 80% less variability of the concentration, dose, and/or potency as compared to a method using qPCR. In certain embodiments, the methods disclosed herein comprising ELISA result in an AAV preparation with about 10% to about 75%, about 10% to about 70%, about 10% to about 65%, about 15% to about 60% , about 15% to about 55%, about 20% to about 50%, about 20% to about 48%, about 20% to about 46%, about 20% to about 44%, about 20% to about 42%, about 20% to about 40%, about 20% to about 38%, about 20% to about 36%, about 20% to about 34%, or about 20% to about 32% less variability of the concentration, dose, and/or potency as compared to a method using qPCR. In certain embodiments, the methods disclosed herein comprising ELISA result in an AAV preparation with about 20% to about 33% less variability of the concentration, dose, and/or potency as compared to a method using qPCR. In certain embodiments, the methods disclosed herein comprising ELISA result in an AAV preparation with at least about 10%, at least about 11%, at least about 12%, at least about 13%, at least about 14%, at least about 15%, at least about 16%, at least about 17%, at least about 18%, at least about 19%, at least about 20%, at least about 21%, at least about 22%, at least about 23%, at least about 24%, at least about 25%, at least about 26%, at least about 27%, at least about 28%, at least about 29%, at least about 30%, at least about 31%, at least about 32%, at least about 33%, at least about 34%, at least about 35%, at least about 36%, at least about 37%, at least about 38%, at least about 39%, at least about 40%, at least about 41%, at least about 42%, at least about 43%, at least about 44%, at least about 45%, at least about 46%, at least about 47%, at least about 48%, at least about 49%, or at least about 50% less variability of the concentration, dose, and/or potency as compared to a method using qPCR.

In all embodiments, the methods comprise testing an AAV preparation via an AAV-specific ELISA. In certain embodiments, the AAV-specific ELISA is sufficient to provide a representative reading on dose or potency of the AAV preparation, because the majority of the capsids in the AAV preparation are full capsids. For example, the percentage of full AAV capsids is a majority from about 50% to about 100%. In certain embodiment, the percentage of full AAV capsids is from about 50% to about 95%, about 50% to about 90%, about 50% to about 85%, about 50% to about 80%, about 55% to about 50%, about 60% to about 80%, or about 65% to about 75%. In certain embodiments, at least about 60%, at least about 70%, at least about 80%, at least about 85%, at least about 90%, at least about 91%, at least about 92%, at least about 93%, at least about 94%, at least about 95%, at least about 96%, at least about 97%, at least about 98%, at least about 99%, or at least about 100% of the AAV capsids are full AAV capsids. In certain embodiments, at least about 60% of the AAV capsids are full AAV capsids. In certain embodiments, the AAV-specific ELISA is sufficient to provide a representative reading on potency of the AAV preparation, because the ratio of full versus empty capsids in the AAV preparation is consistent from batch to batch. In certain embodiments, consistent refers to a coefficient of variation (CV) of about 0% to about 15%, about 2% to about 12%, about 5% to about 10%, about 7% to about 9%. In certain embodiments, consistent refers to a CV of about 15% or less, about 12% or less, about 10% or less, about 9% or less, about 8% or less, about 7% or less, about 6% or less, about 5% or less, about 4% or less, about 3% or less, about 2% or less, or about 1% or less. In certain embodiments, consistent refers to a CV of about 1% or less. In certain embodiments, the AAV-specific ELISA in combination with CryoTEM is required to test the AAV preparation potency.

In all embodiments, the methods comprise testing an AAV fraction or preparation via CryoTEM. In certain embodiments, the CryoTEM is sufficient to provide a representative reading on potency of the AAV preparation. In certain embodiments, the CryoTEM in combination with AAV-specific ELISA is required to test the AAV preparation potency.

The AAV preparation produced and/or measured via the methods of the present disclosure may be suitable for administration to a subject. Applicable AAV vectors are described in more detail below. The AAV preparation produced through the methods of the present disclosure may be sterile and/or of good manufacturing practice (GMP) grade. The AAV preparation produced through the methods of the present disclosure may conform to the requirements set forth in the U.S. Pharmacopeia Chapter 1046 or the European Pharmacopoeia on gene therapy medicinal products or as mandated by the U.S. Food and Drug Administration (USFDA) or the European Medicines Agency (EMA).

As used herein, the methods can be used on an AAV fraction and/or AAV preparation. The term "preparation" and "fraction" may be used interchangeably in this application. In certain embodiments, the term "fraction" can mean an AAV sample or batch that can be used to generate a final AAV "preparation" that will be administered to a subject. For example, the AAV fraction may undergo additional processing, purification, or volume adjustment before forming the final AAV preparation.

### Quantitative and/or Qualitative Methods

The methods of the present disclosure comprise one or more quality control steps, *e.g.,* steps to measure the concentration, dose, and/or potency of the AAV fractions or preparations obtained after one or more steps (e.g., after each step) of the purification process, including the final step for making a preparation for administration.

The methods of the present disclosure comprise an ELISA assay, specific for AAV (e.g., AAV antigen), for quantitating the number of AAV capsids. In certain embodiments, the ELISA assay can include, but is not limited to, an immunosorbent assay, direct ELISA, indirect ELISA, sandwich ELISA, and/or a competitive ELISA. In certain embodiments, the ELISA is a sandwich ELISA. A non-limiting example of an ELISA assay is presented in Example 1.

In certain embodiments, the AAV antigen is an AAV1, AAV2, AAV3, AAV4, AAV5, AAV6, AAV7, AAV8, AAV9, AAV10, or a chimeric AAV antigen. In certain embodiments, the AAV antigen is an AAV8 antigen. In certain embodiments, the AAV antigen is from a recombinant AAV (rAAV). In certain embodiments, the AAV antigen is from a genetically engineered AAV, or chemically modified AAV, or both.

In certain embodiments, the ELISA comprises an antibody specific for an AAV epitope. In certain embodiments, the AAV epitope is a conformational epitope present on assembled AAV capsids. In certain embodiments, the AAV epitope is a linear epitope present on assembled AAV capsids. Examples of such epitopes includes, but is not limited to, capsid virion proteins VP1, VP2, and/or VP3. In certain embodiments, the AAVs are genetically engineered to express additional virion proteins on the surface of the capsid, and those engineered proteins can be used/detected in an ELISA assay. In certain embodiments, the AAV are chemically modified to express variants of virion protein, which can be used/detected in an ELISA assay (e.g., VP1', VP2', VP3', etc...). In certain embodiments, the antibodies identify serotype specific capsid virion proteins.

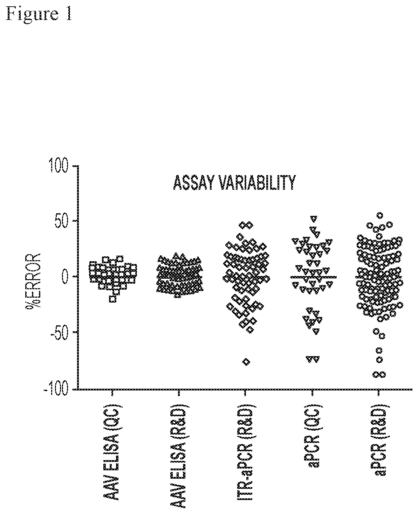

The ELISA may replace qPCR as a way to determine the dose and/or potency of an AAV fraction or preparation. As demonstrated in Figure 1, the ELISA technique of the invention has significantly less variability than the qPCR method. In certain embodiments, the methods of the present disclosure comprise evaluating an AAV fraction or preparation via an AAV-specific ELISA. In certain embodiments, all the methods disclosed herein do not include a qPCR step.

In certain embodiments, the methods of the present disclosure comprise testing an AAV fraction or preparation obtained after ultracentrifugation via an AAV-specific ELISA to determine the number of AAV capsids in the AAV fraction or preparation. In certain embodiments, the methods of the present disclosure comprise testing an AAV fraction or preparation obtained after depth filtration via an AAV-specific ELISA to determine the number of AAV capsids in the AAV fraction or preparation. In certain embodiments, the methods of the present disclosure comprise testing an AAV fraction or preparation obtained after concentrating an AAV fraction or preparation using an ultra-/ diafiltration system via an AAV-specific ELISA to determine the number of AAV capsids in the AAV fraction or preparation. In certain embodiments, the methods of the present disclosure comprise testing an AAV fraction or preparation obtained after a tangential flow filtration (TFF) step via an AAV-specific ELISA to determine the number of AAV capsids in the AAV fraction or preparation. In certain embodiments, the methods of the present disclosure comprise testing an AAV fraction or preparation obtained after negative anion exchange (AEX) chromatography via an AAV-specific ELISA to determine the number of AAV capsids in the AAV fraction or preparation. In certain embodiments, the methods of the present disclosure comprise testing an AAV fraction or preparation obtained after a polish step via an AAV-specific ELISA to determine the number of AAV capsids in the AAV fraction or preparation. In certain embodiments, the methods of the present disclosure comprise testing a purified AAV preparation via an AAV-specific ELISA to determine the number of AAV capsids in the AAV preparation.

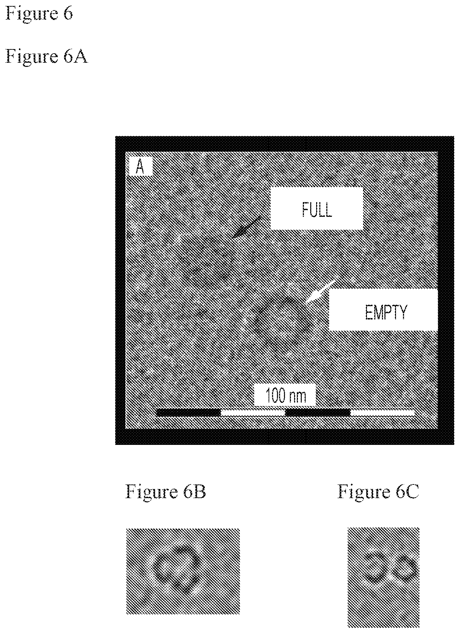

In all embodiments, the methods of the present disclosure comprise measuring full versus empty AAV capsids (e.g., percentage of ratio of full versus empty AAV capsids). In all embodiments, the method for measuring full versus empty AAV capsids is CryoTEM (see Figure 6). In all embodiments, the method entails using both CryoTEM and an AAV-specific ELISA as described above. In certain embodiments, the ELISA is a sandwich ELISA. In certain embodiments, the sandwich ELISA comprises an antibody specific for an AAV epitope. In certain embodiments, the AAV epitope is a conformational epitope present on assembled AAV capsids. A non-limiting method of measuring the amount of full versus empty capsids via CryoTEM is described herein as Example 2.

One advantage of using CryoTEM is that there is no need for a negative stain. CryoTEM also results in the ability to quantitate the amount of full AAV capsids. In certain embodiments, use of CryoTEM results in not overestimating the full AAV capsid amount due to a false positive signal.

The methods of the present disclosure comprise a method evaluating the number or a percentage of full versus empty AAV capsids. In all embodiments, the methods comprise CryoTEM. In certain embodiments, the methods comprise: (i) embedding an AAV fraction or preparation in a substrate in an inert support; (ii) flash-freezing the embedded AAV fraction or preparation; (iii) imaging the embedded AAV fraction or preparation using cryogenic transmission electron microscopy; and (iv) quantitating the percentage of full AAV capsids versus empty AAV capsids.

For step (i), examples of suitable substrates for use in the method includes, but is not limited to amorphous, non-crystalline ice. Examples of suitable inert support for use in the method includes, but is not limited to a film of carbon, thermoplastic resins, and polyvinyl formals (e.g., polymers formed from polyvinyl alcohol and formaldehyde as copolymers with polyvinyl acetate, such as, but not limited to, Formvar or Vinylec, polyvinyl formal stabilized with carbon, silicon monoxide on polyvinyl formal, pure carbon film, carbon type-A: carbon support films (e.g., removable polyvinyl formal on the opposite side of the grid), carbon type-B: polyvinyl formal film (e.g., coated with a heavier layer of carbon), and silicon monoxide on carbon type-A). In certain embodiments, step (i) above involves deposition of a sample onto a thin supporting film of carbon in a temperature (e.g., (-196 °C) or below) and humidity controlled environment. In certain embodiments, the humidity level can be relative humidity. For step (ii), a sample can be flash-frozen. In certain embodiments, flash freezing can entail using liquid ethane, liquid nitrogen, liquid propane, or helium near liquid nitrogen temperature. For example, the container of liquid ethane, liquid nitrogen, liquid propane, or helium is surrounded by liquid nitrogen.

In certain embodiments, the AAV fraction or preparation is frozen so rapidly (e.g., at 104 to 106 K per second) that ice crystals are unable to form. In certain embodiments, amorphous ice is produced either by rapid cooling of liquid water or by compressing ordinary ice at low temperatures. In certain embodiments, after excess of AAV fraction or preparation is removed leaving some of the specimen adhered, the grid is vitrified in liquid ethane and then stored in liquid nitrogen. For discussion of additional steps to perform the CryoTEM, see Cabra and Samso, J. Visualized Experiments, (2015) 95(e52311):1-11).

CryoTEM analysis of AAV particles can be utilized to assess the overall specimen morphology, i.e. presence of various AAV morphologies (generally including spherical and deformed AAV particles, subunit structures and larger structurally less defined morphologies). Figure 6A provides an example of how one can visually discern "full" versus "empty" AAV particle. For example, the full capsid displays an inner density with no distinct boundary between the shell and the core. AAV capsids displaying a distinct outer shell and minute internal density are classified as empty capsids.

CryoTEM can also be used to assess uncertain capsids. Figure 6B and 6C provide examples of deformed/uncertain capsids which would not be included as full capsids. For example, CryoTEM can be used to count "full", "empty", and "uncertain" capsids.

CryoTEM can also be used to determine the level of packaging of the particles by either manually classifying particles or using an automated image analysis methodology.

In certain embodiments, the methods of the present disclosure comprise testing an AAV fraction or preparation obtained after ultracentrifugation via CryoTEM to determine the quantity and/or quality of the AAV capsids in the AAV fraction or preparation. In certain embodiments, the methods of the present disclosure comprise testing an AAV fraction or preparation obtained after depth filtration via CryoTEM to determine the quantity and/or quality of the AAV capsids in the AAV fraction or preparation. In certain embodiments, the methods of the present disclosure comprise testing an AAV fraction or preparation obtained after concentrating an AAV fraction or preparation using an ultra-/ diafiltration system via CryoTEM to determine the quantity and/or quality of the AAV capsids in the AAV fraction or preparation. In certain embodiments, the methods of the present disclosure comprise testing an AAV fraction or preparation obtained after a tangential flow filtration (TFF) step via CryoTEM to determine the quantity and/or quality of the AAV capsids in the AAV fraction or preparation. In certain embodiments, the methods of the present disclosure comprise testing an AAV fraction or preparation obtained after negative anion exchange (AEX) chromatography via CryoTEM to determine the quantity and/or quality of the AAV capsids in the AAV fraction or preparation. In certain embodiments, the methods of the present disclosure comprise testing an AAV fraction or preparation obtained after a polishing step via CryoTEM to determine the quantity and/or quality of the AAV capsids in the AAV fraction or preparation. In certain embodiments, the methods of the present disclosure comprise testing a purified AAV preparation via CryoTEM to determine the quantity and/or quality of the AAV capsids in the AAV fraction or preparation.

In certain embodiments, the methods of the present disclosure comprise testing an AAV fraction or preparation obtained after ultracentrifugation via an AAV-specific ELISA and CryoTEM to determine the quantity and quality of the AAV capsids in the AAV fraction or preparation. In certain embodiments, the methods of the present disclosure comprise testing an AAV fraction or preparation obtained after depth filtration via an AAV-specific ELISA and CryoTEM to determine the quantity and quality of the AAV capsids in the AAV fraction or preparation. In certain embodiments, the methods of the present disclosure comprise testing an AAV fraction or preparation obtained after concentrating an AAV fraction or preparation using an ultra-/ diafiltration system via an AAV-specific ELISA and CryoTEM to determine the quantity and quality of the AAV capsids in the AAV fraction or preparation. In certain embodiments, the methods of the present disclosure comprise testing an AAV fraction or preparation obtained after a tangential flow filtration (TFF) step via an AAV-specific ELISA and CryoTEM to determine the quantity and quality of the AAV capsids in the AAV fraction or preparation. In certain embodiments, the methods of the present disclosure comprise testing an AAV fraction or preparation obtained after negative anion exchange (AEX) chromatography via an AAV-specific ELISA and CryoTEM to determine the quantity and quality of the AAV capsids in the AAV fraction or preparation. In certain embodiments, the methods of the present disclosure comprise testing an AAV fraction or preparation obtained after a polishing step via an AAV-specific ELISA and CryoTEM to determine the quantity and quality of the AAV capsids in the AAV fraction or preparation. In certain embodiments, the methods of the present disclosure comprise testing a purified AAV preparation via an AAV-specific ELISA and CryoTEM to determine the quantity and quality of the AAV capsids in the AAV fraction or preparation.

Analytical Ultracentrifugation (AUC) is capable of separating proteins according to the sedimentation coefficient. For identical proteins, the sedimentation coefficient can be correlated to aggregation status. Briefly, samples are diluted with the corresponding sample buffer and then transferred to cell assemblies with built-in quartz windows and loaded in the rotor, which is then rotated at a constant speed. Protein molecules of different size migrate at different sedimentation speed towards the bottom of the cell and are monitored continuously during centrifugation by UV detection at 280 nm. The collected data set allows for a computational analysis, which deconvolutes the sedimentation and diffusion processes, resulting in a differential sedimentation coefficient distribution c(s). This resolves the different species of the sample, and presents their s-values and populations. The distribution of sedimentation coefficients is integrated and relative area percentages as well as S-values of the peak maxima are given as results of the analysis.

### AAV Preparation

In certain embodiments, the method for producing an AAV preparation, comprising: (i) transfecting host cells with at least one plasmid comprising the gene of interest; (ii) collecting supernatant or cell suspension of a cell culture comprising AAV capsids to create an AAV fraction or preparation; (iii) quantifying the total number of AAV capsids in the AAV fraction or preparation using an AAV-specific ELISA assay; and (iv) preparing an AAV preparation with a desired concentration based on the total number of AAV capsids determined in step (iii). In certain embodiments, the method comprises concentrating the AAV fraction. In certain embodiments, the method comprises removing at least a portion of empty capsids from the AAV fraction. In certain embodiments, the amount of empty capsids are removed to create an AAV fraction or preparation with a specific concentration of full capsids and/or a specific ratio of full:empty capsids. In certain embodiment, the AAV fraction or preparation is diluted with the appropriate buffer to the desired dose.

In all embodiments, the method comprises evaluating or confirming the percentage or ratio of full versus empty (full:empty) AAV capsids by CryoTEM. In certain embodiments, the dose and/or potency is not determined by qPCR. In certain embodiments, the percentage of full AAV capsids is about 40% to about 100%. In certain embodiment, the percentage of full AAV capsids is from about 40% to about 95%, about 40% to about 90%, about 40% to about 85%, about 40% to about 80%, about 45% to about 75%, about 50% to about 70%, or about 55% to about 65%. In certain embodiments, the percentage of full AAV capsids is between about 60% to about 80%. In certain embodiments, at least about 60% of the AAV capsids are full AAV capsids. In certain embodiments, at least about 40%, at least about 50%, at least about 60%, at least about 70%, at least about 80%, at least about 85%, at least about 90%, at least about 91%, at least about 92%, at least about 93%, at least about 94%, at least about 95%, at least about 96%, at least about 97%, at least about 98%, at least about 99%, or at least about 100% of the AAV capsids are full AAV capsids. In certain embodiments, the methods as disclosed herein are used to generate AAV fractions or preparations with consistent amounts of full capsids between AAV fractions or preparation.

In certain instances disclosed herein, the concentration of the AAV preparations is between about 1×10¹⁰ cp/ml to about 1×10²⁰ cp/ml. In certain embodiments, for the methods, fractions, or preparations disclosed herein, the concentration of the AAV preparations is between about 1×10¹¹ cp/ml to about 1×10¹⁹ cp/ml, about 1×10¹² cp/ml to about 1×10¹⁸ cp/ml, about 1×10¹³ cp/ml to about 1×10¹⁷ cp/ml, or about 1×10¹⁴ cp/ml to about 1×10¹⁶ cp/ml. In certain embodiments, for the methods and preparations disclosed herein, the concentration of the AAV preparation is between about 1×10¹² cp/ml to about 1×10¹⁵ cp/ml, about 1×10¹³ cp/ml to about 1×10¹⁵ cp/ml, or about 1×10¹² cp/ml to about 1×10¹³ cp/ml. In certain embodiments, for the methods and preparations disclosed herein, the concentration of the AAV preparation is between about 1×10¹⁴ cp/ml to about 5×10¹⁴ cp/ml, about 2×10¹⁴ cp/ml to about 3×10¹⁴ cp/ml, or about 3.5×10¹⁴ cp/ml to about 5×10¹⁵ cp/ml. In certain embodiments, cp/ml can be total capsids per ml or full capsids per ml. In certain embodiments, the cp/ml is total capsids per ml.

In certain embodiments, the AAV preparation comprises at least about 10¹² virus particles (vp) produced from about 1000 L of starting material (e.g., cell culture) or at least about 10¹³ virus particles (vp) produced from about 1000 L of starting material (e.g., cell culture).

In certain embodiments, for the methods or preparations disclosed herein, the dose of the AAV preparation is between about 1 x 10⁵ cp/kg to about 1 x 10²⁵ cp/kg. In certain embodiments, for the methods or preparations disclosed herein, the dose of the AAV preparation is between about 1 x 10⁶ cp/kg to about 1 x 10²⁴ cp/kg, about 1 x 10⁷ cp/kg to about 1 x 10²¹ cp/kg , about 1 x 10⁸ cp/kg to about 1 x 10²² cp/kg, about 1 x 10⁹ cp/kg to about 1 x 10²¹ cp/kg, about 1 x 10¹⁰ cp/kg to about 1 x 10²⁰ cp/kg, about 1 x 10¹¹ cp/kg to about 1 x 10¹⁹ cp/kg, about 1 x 10¹² cp/kg to about 1 x 10¹⁸ cp/kg, about 1 x 10¹³ cp/kg to about 1 x 10¹⁷ cp/kg, or about 1 x 10¹⁴ cp/kg to about 1 x 10¹⁶ cp/kg. In certain embodiments, for the methods and preparations disclosed herein, the dose of the AAV preparation is between about 1 x 10¹² cp/kg to about 1 x 10²⁰ cp/kg, about 1 x 10¹³ cp/kg to about 1 x 10¹⁹ cp/kg, about 1 x 10¹⁴ cp/kg to about 1 x 10¹⁸ cp/kg, or about 1 x 10¹⁵ cp/kg to about 1 x 10¹⁷ cp/kg. In certain embodiments, for the methods and preparations disclosed herein, the dose of the AAV preparation is between about 1 x 10¹⁰ cp/kg to about 1 x 10¹⁶ cp/kg. In certain embodiments, for the methods and preparations disclosed herein, the dose of the AAV preparation is at least about 1 x 10⁶ cp/kg, at least about 1 x 10⁷ cp/kg, at least about 1 x 10⁸ cp/kg, at least about 1 x 10⁹ cp/kg, at least about 1 x 10¹⁰ cp/kg, at least about 1 x 10¹¹ cp/kg, at least about 1 x 10¹² cp/kg, at least about 1 x 10¹³ cp/kg, at least about 1 x 10¹⁴ cp/kg, at least about 1 x 10¹⁵ cp/kg, at least about 1 x 10¹⁶ cp/kg, at least about 1 x 10¹⁷ cp/kg, at least about 1 x 10¹⁸ cp/kg, at least about 1 x 10¹⁹ cp/kg, at least about 1 x 10²⁰ cp/kg, at least about 1 x 10²¹ cp/kg, at least about 1 x 10²² cp/kg, at least about 1 x 10²³ cp/kg, at least about 1 x 10²⁴ cp/kg, or at least about 1 x 10²⁵ cp/kg. In certain embodiments, cp/kg can be total capsids per kg of the subject or full capsids per kg of the subject. In certain embodiments, the cp/kg is total capsids per kg of the subject.

In certain embodiments, the AAV preparation of the present disclosures is highly pure, highly potent and suitable for clinical use in a subject. In certain embodiments, the AAV preparation comprises AAV capsid particles of a homogenous population and high purity. In certain embodiments, the AAV preparation comprises full-length vector DNA. In exemplary embodiments, the AAV preparation is substantially free of unwanted contaminants, including but not limited to, AAV capsid particles containing truncated or incomplete vector DNA, AAV particles with incomplete protein composition and oligomerized structures, or contaminating viruses, e.g., non AAV, lipid enveloped viruses. In exemplary embodiments, the AAV preparation contains a high amount of encoding cDNA of the protein of interest. In certain embodiments, the AAV preparation of the present disclosure is suitable for administration to a subject. In certain embodiments, the AAV preparation is sterile and/or of good manufacturing practice (GMP) grade. In certain embodiments, the AAV preparation conforms to the requirements set forth in the U.S. Pharmacopeia Chapter 1046 or the European Pharmacopoeia on gene therapy medicinal products or as mandated by the U.S. Food and Drug Administration (USFDA) or the European Medicines Agency (EMA). In certain embodiments, the AAV preparation is a ready-to-use preparation for direct administration to a subject with little to no processing or handling.

The terms "patient" and "subject" are used interchangeably and are used in their conventional sense to refer to a living organism suffering from or prone to a condition that can be prevented or treated by administration of a composition of the present disclosure, and includes both humans and non-human animals. Examples of subjects include, but are not limited to, humans, chimpanzees and other apes and monkey species; farm animals such as cattle, sheep, pigs, goats and horses; domestic mammals such as dogs and cats; laboratory animals including rodents such as mice, rats and guinea pigs; birds, including domestic, wild and game birds such as chickens, turkeys and other gallinaceous birds, ducks, geese, and the like. The term does not denote a particular age. Thus, adult, juvenile and newborn individuals are of interest.

### Source of AAV

With regard to the methods of the present disclosure, the AAV may be of any AAV serotype. In certain embodiments, the AAV described herein are of AAV1 serotype, AAV2 serotype, AAV3 serotype, AAV4 serotype, AAV5 serotype, AAV6 serotype, AAV7 serotype, AAV8 serotype, AAV9 serotype, AAV10 serotype, or chimeric AAV vectors. In certain embodiments, the AAV is wild type. In certain embodiments, the AAV is a recombinant AAV (rAAV). In certain embodiments, the AAV is modified by genetic engineering and/or is chemically modified. In certain embodiments, the AAV comprises a modified capsid, e.g., a genetically engineered or a chemically-modified AAV capsid. In certain embodiments, the AAV is of the AAV8 serotype. In certain embodiments, the AAV is of the AAV9 serotype.

With regard to the methods of the invention, the AAV fraction is in exemplary aspects a concentrated AAV fraction. In certain embodiments, the AAV fraction comprises at least about 1 x 10¹⁰, about 1 x 10¹¹, about 1 x 10¹² , about 1 x 10¹³, about 1 x 10¹⁴ , about 1 x 10¹⁵, or about 1 x 10¹⁶, AAV total capsids per mL. In certain embodiments, the AAV fraction comprises at least about 1 x 10¹² AAV total capsids per mL. The AAV capsids may include empty AAV capsids and full AAV capsids.

In certain embodiments, the AAV represents an AAV fraction or preparation produced by transfected host cells. In certain embodiments, the AAV fraction or preparation represents a supernatant harvested or cell suspension from a cell culture comprising host cells transfected with a triple plasmid system, wherein one plasmid of the system comprises a gene or cDNA of interest, one plasmid encodes capsid protein VP1, capsid protein VP2 and/or capsid protein VP3. In certain embodiments, VP1, VP2, and/or VP3 are AAV8 VP1, VP2, and/or VP3. Triple plasmid transfection for purposes of rAAV production is known in the art. See, e.g., Qu et al., *2015, supra,* and Mizukami et al., "A Protocol for AAV vector production and purification." PhD dissertation, Division of Genetic Therapeutics, Center for Molecular Medicine, 1998; and Kotin et al., Hum Mol Genet 20(R1): R2-R6 (2011). In certain embodiments, the transfection may be carried out using inorganic compounds, e.g., calcium phosphate, or organic compounds, polyethyleneimine (PEI), or non-chemical means, e.g., electroporation.

In certain embodiments, the host cells are adherent cells. In certain embodiments, the host cells are suspension cells. In certain embodiments, the host cells are HEK293 cells or Sf9 cells (e.g., baculovirus infected Sf9 cells) or HeLa or BHK (Herpes Virus System). In certain embodiments, the cell culture comprises culture medium which is serum and protein free. In certain embodiments, the medium is chemically defined and is free of animal derived components, e.g., hydrolysates.

In certain embodiments, the fraction comprising rAAV particles represents a fraction comprising HEK293 cells transfected with a triple plasmid system. In certain embodiments, the fraction comprising AAV particles represents a fraction of the harvest after about 2 to about 7 days after transfection of the HEK293 cells or when the cell culture has a cell density of greater than or about 5×10⁶ cells/mL and has a cell viability of greater than or about 50%.

In certain embodiments, the AAV is prepared by a triple plasmid transfection followed by harvest from one to 7 days later. In certain embodiments, the AAV is prepared from cell disruption.

In certain embodiments, the AAV is prepared by the following: The HEK293 cells are adherent and grown in a commercially-available culture medium that may be chemically-defined and may be free of animal-derived components, e.g. serum and proteins. The cells are cultured to a cell density of about 3 x 10⁶ to about 12 x 10⁶ cells/ml, e.g., about 6 × 10⁶ to about 10 × 10⁶ cells/ml. The cells are then split in about a 1:2 ratio such that the cell density is about 3 - 5 x 10⁶ cells/ml. After the split, the cells may be transfected with three plasmids that include (1) a helper plasmid capable of providing one or more helper viral functions essential AAV production, (2) a plasmid that encodes for one or more genes involved in capsid generation, replication and packaging of the virus, and (3) a plasmid comprising a gene of interest (GOI) to be packaged into the resulting rAAV particle. For example, the GOI may be a vector DNA comprising human coagulation Factor IX Padua in a single stranded self-complementary form, with the vector DNA. As another example, the GOI may be a vector DNA comprising human coagulation Factor IX Padua in a double stranded self-complementary form, with the vector DNA having a full length of 4.8 kB. As another example, the GOI may be a vector DNA comprising a B-domain deleted human coagulation Factor VIII in a single stranded self-complementary form, with the vector DNA having a full length of 4.8 kB. Other GOI may be used. Transfection may be carried out in a transient manner, such as by using cationic polymers. Before elution, the HEK293 cell line may be cultivated for at least about 1 days, e.g., 3-5 days, before harvesting.

In certain embodiments, the fraction comprising AAV particles is described in U.S. Provisional Application No. 62/417,775 and the publication of International Application No. PCT/US2017/059967.

### Purification Steps

In exemplary embodiments, the methods of the present disclosure comprise an ultracentrifugation step during which a density gradient is formed. Though not wishing to be bound to a theory, it is believed that the ultracentrifugation step allows for full AAV capsids to be separated from empty AAV capsids. Examples of ultracentrifugation protocols can be found in, for example, WO2008135229 and the publication of PCT/US2017/059967.

The methods of the present disclosure may comprise yet other additional steps, which may further increase the purity of the AAV and remove other unwanted components and/or concentrate the fraction and/or condition the fraction for a subsequent step.

In certain embodiments, the method comprises a depth filtration step. In certain embodiments, the method comprises subjecting a fraction of a transfected HEK293 cell culture supernatant to depth filtration using a filter comprising cellulose and perlites and having a minimum permeability of about 500L/m². In certain embodiments, the method further comprises use of a filter having a minimum pore size of about 0.2 µm. In certain embodiments, the depth filtration is followed by filtration through the filter having a minimum pore size of about 0.2 µm. In certain embodiments, one or both of the depth filter and filter having a minimum pore size of about 0.2 µm are washed and the washes are collected. In certain embodiments, the washes are pooled together and combined with the filtrate obtained upon depth filtration and filtration with the filter having a minimum pore size of about 0.2 µm. In certain embodiments, the depth filtration step and other filtration step occurs prior to the ultracentrifugation step described herein.

In certain embodiments, the methods of the present disclosure comprise one or more chromatography steps. In certain embodiments, the methods comprise a negative chromatography step whereby unwanted components bind to the chromatography resin and the desired AAV does not bind to the chromatography resin. In certain embodiments, the methods comprise a negative anion exchange (AEX) chromatography step, or an AEX chromatography step in the "non-binding mode". Advantages of "non-binding mode" include relative ease of carrying out the procedure and in conducting subsequent assaying. Accordingly, in certain embodiments, the methods of purifying AAV particles comprise performing negative anion exchange (AEX) chromatography on a fraction comprising AAV particles by applying the fraction to an AEX chromatography column or membrane under conditions that allow for the AAV to flow through the AEX chromatography column or membrane and collecting AAV particles. In certain embodiments, the fraction is applied to the AEX chromatography column or membrane with a loading buffer comprising about 100 mM to about 150 mM salt, e.g., NaCl, optionally, wherein the pH of the loading buffer is about 8 to about 9. In certain embodiments, the loading buffer comprises about 115 mM to about 130 mM salt, e.g., NaCl, optionally, wherein the loading buffer comprises about 120 mM to about 125 mM salt, e.g., NaCl. In certain embodiments, the negative AEX step occurs prior to the ultracentrifugation step described herein.

In certain embodiments, the methods of the present disclosure comprise concentrating an AAV fraction using an ultra/diafiltration system. In certain embodiments, the methods of the present disclosure comprise one more tangential flow filtration (TFF) steps. In certain embodiments, the AAV fraction undergoes ultra-/dia-filtration. In certain embodiments, the AAV fraction is concentrated with the ultra/diafiltration system before a step comprising performing negative AEX chromatography, after a step comprising performing negative AEX chromatography, or before and after comprising performing negative AEX chromatography.

In certain embodiments, the methods of the present disclosure comprise treating a fraction comprising AAV particles with a solvent detergent to inactivate lipid enveloped viruses.

In certain embodiments, the methods of the present disclosure comprise filtration of a fraction comprising rAAV particles to remove viruses of greater size than the rAAV particles in the fraction. In certain embodiments, the method of the present disclosure comprises filtration of a fraction comprising AAV to remove viruses sized greater than or about 35 nm. In certain embodiments, the pore size of the filter is in the nanometer range, and, in certain embodiments, the method comprises nanofiltration. In certain embodiments, the method of the present disclosure comprises use of a nanofilter of pore size in the range of 35 nanometer ±2 nanometer, as determined by a water flow method. Classification of the type of filter is dependent on membrane structure, material, and vendor.

In certain embodiments, during the filtration step, a pressure difference over the filter is maintained. In certain embodiments, the pressure (pressure drop across the filter) is about 0.02 MPa to about 0.1 MPa. In certain embodiments, the pressure (e.g., pressure drop across the filter) is about 0.02 MPa to about 0.08 MPa. In case the filter is run in dead-end mode, the pressure difference can be affected by the feed pressure of the sample applied (i.e., by adjustment of a pump to a specific flow, which affects the feed pressure).

In certain embodiments, the filtration step for removal of viruses larger than the rAAV particles occurs once during the process of the present disclosure. In certain embodiments, the filtration step occurs twice during the process. In certain embodiments, the filtration step for removal of viruses larger than the rAAV particles occurs after the ultracentrifugation step described herein. In certain embodiments, the filtration step for removal of viruses larger than the rAAV particles occurs after a polish step.

In certain embodiments, the methods of the present disclosure comprise a polish step comprising performing AEX chromatography, optionally with a column comprising tentacle gel.

The use of the terms "a" and "an" and "the" and similar referents in the context of describing the disclosure (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted.

Recitation of ranges of values herein are merely intended to serve as a shorthand method of referring individually to each separate value falling within the range and each endpoint, unless otherwise indicated herein, and each separate value and endpoint is incorporated into the specification as if it were individually recited herein.

The invention is defined by the appended claims.

The following examples are given merely to illustrate the present invention and not in any way to limit its scope. The invention is strictly limited to what is claimed.

### EXAMPLES

### EXAMPLE 1

This example demonstrates an AAV-specific ELISA. The ELISA determines the amount of AAV8 particles present in the tested fraction or preparation.

An AAV-specific ELISA was carried out to identify AAV-containing fractions. A conformation-specific monoclonal antibody is used for the detection of an epitope, repeatedly expressed on assembled AAV-8 particles.

AAV8 ELISA was carried out with an AAV-8 titration ELISA Kit (Art. No. PRAAV8; Progen (Heidelberg, Germany)) on a TECAN Roboter system. Briefly, a monoclonal antibody specific for a conformational epitope on assembled AAV8 capsids (ADK8) was coated onto microtiter strips and was used to capture AAV8 particles from the AAV fraction. The capture AAV8 particles were detected by two steps. In a first step, a biotin-conjugated monoclonal antibody specific for the ADK8 antibody was bound to the immune complex (of ADK8 and an ADK8 antibody). Streptavidin peroxidase conjugates were added to the immune complexes bound to the biotin-conjugated monoclonal antibody and the streptavidin peroxidase conjugates reacted with the biotin. An AAV2/8 particle preparation, WL217S, containing labelled AAV-8 particles at set concentrations, was used as the standard. A peroxidase substrate solution was added resulting in a color reaction proportional to the amount of bound AAV capsid particles. The color reaction was measured photometrically at 450 nm.

The pre-coated plate was filled with 100 µL/well dilution buffer. Then the dilution series for the assay standard, the assay control and the samples were prepared directly on the plate. The dissolved assay standard was diluted in duplicates six times 1+1 directly on the plate by mixing 100 µL with 100 µL dilution buffer already present in the wells, while the assay control was diluted 1/200 before the six serial dilutions were prepared directly on the plate. For the samples, two serial dilutions were prepared in independent duplicates. The plate was then incubated 60 ± 10 min at +37°C (set point) on the microplate shaker, turning the plate by 180° after 30 ± 5 min. After a washing step (washing buffer: Phosphate-buffered saline (PBS) with Tween 20, 0.8% NaCl, 0.02% KCl, 0.02% KH₂PO₄, 0.126% Na₂HPO₄×2H₂O, 0.05% Tween 20, pH neutral (checked with indicator paper), 100 µL/well detection antibody was added and the plate was incubated 30 ± 5 min after addition. This incubation was terminated by a washing step. Then, 100 µL/well tetramethylbenzidine substrate was added. The color reaction was allowed to proceed at room temperature for 25 ± 5 min, before it was stopped by the addition of stopping solution. The plate was measured within 30 min using the ELISA reader at 450 nm and 620 nm as reference wavelength. The calibration curve was then obtained after log-log fitting.

### EXAMPLE 2

This example demonstrates the cryogenic transmission electron microscopy (CryoTEM).

CryoTEM enables visualization of nanosized particles such as AAV. The technique involves deposition of an AAV preparation onto a thin supporting film of carbon in a temperature and humidity controlled environment. After removal of excess of the AAV preparation leaving some of the AAV preparation adhered, the grid was vitrified in liquid ethane and then stored in liquid nitrogen. CryoTEM analysis of AAV capsid particles was utilized to assess the overall specimen morphology, i.e. presence of various AAV morphologies (generally including spherical and deformed AAV particles, subunit structures and larger structurally less defined morphologies), and the level of packaging of the particles by an automated image analysis methodology.

AAV fraction or preparation were preserved close to their native state by embedding them in amorphous, non-crystalline ice on an inert support by flash-freezing under cryogenic conditions. Image analysis based was done using an automated, objective analysis method using Vironova Analyzer Software (VAS).

AAV capsid particles displaying an inner density with no distinct boundary between the shell and the core are classified as filled particles (Figure 6A). AAV capsid particles displaying a distinct outer shell and minute internal density are classified as empty particles (Figure 6A). Uncertain particles, include AAV capsids in the form of a trimeric structure (Figure 6B), which could be empty or full, and broken AAV capsids (Figure 6C).

The CryoTEM determines the amount of complete full versus empty capsids in AAV preparations.

### EXAMPLE 3

This example demonstrates that use of AAV-specific ELISA results in less variability than the use of a qPCR technique.

**AAV8 ELISA:** Total AAV8 capsid particle titer was measured using a commercial kit (Progen) calibrated to the ATCC^{®} AAV8 reference standard material for capsid particle (cp) concentrations as described in Example 1.

**qPCR:** A quantitative PCR assay based on Taqman^{®} chemistry was developed to measure vector genome copy concentrations of the transgene DNA. For ITR-qPCR for FVIII, the vector genome titer (vg) per milliliter (ml) was determined using a TaqMan based qPCR with primers and a fluorescently labeled probe detecting a sequence within the ITR sequences of the vector genome. For detecting the ITR-specific sequence in the AAV particle the samples were treated with DNAse I and, after a subsequent Proteinase K step, the scAAV genome was released from the capsid. Finally, restriction enzyme digest was performed to resolve AAV ITR T-shape structures.

The plasmid used as a reference was linearized with a single cutter restriction enzyme and further purified from an agarose Gel. UV A260 Absorbance of Gel extracted DNA was measured three times in a UV spectrophotometer and the mean value of DNA concentration was calculated in µg per ml.

To determine the copy numbers per ml for the linearized standard plasmid, the molecular weight of ds DNA was calculated in gram per mol considering the exact mass for each individual nucleotide of the underlying sequence.

The vector genome titer in the test article (in vector genomes per mL) was calculated via plasmid standard curve fitting with linear regression.

**CryoTEM:** The samples were preserved close to their native state by embedding them in amorphous, non-crystalline ice on an inert support by flash-freezing under cryogenic conditions as described in Example 2.

**Gel electrophoresis:** Electrophoresis was performed as described (Fagone et al., 2012). Briefly, AAV samples were treated for10 minutes @ 75°C in presence of 0.5% SDS (Sodiumdodecylsulfate) and then cooled down to room temperature. The samples (approximately 1.5E10 vector genomes (vg)/lane) were then loaded onto a 1% 1xTAE agarose gel and separated under an electric field (60 min at 7 V/cm of gel length). The gel was then stained in 2x GelRed (Biotium) solution and imaged by ChemiDocTMMP (Biorad).

***In vivo* biopotency:** An AAV-specific *in vivo* biopotency assay was conducted using an AAV8 preparation evaluated using the antigen ELISA assay to determine the capsid particles per ml - cp/ml. Samples were shipped to the testing facility (frozen, ≤-60°C), thawed and diluted with the formulation buffer to 4×10¹¹cp/ml. The *in vivo* biopotency testing includes up to eight male FVIII knockout mice per group ((B6;129S4-F8 tm2Kaz); Charles River GmbH, Sulzfeld, Germany). The animals received a single intravenous bolus injection of 4×10¹² cp/kg of the test or control samples, shortly after the samples were thawed. The test samples were AAV8-FVIII gene therapy vectors. Collected mouse citrate plasma is used to evaluate the FVIII activity using a FVIII activity assay. The FVIII activity assay is carried out in the presence of calcium ions and phospholipids. Factor X is activated to Factor Xa by Factor IXa. This activation is stimulated by Factor VIII which acts as a cofactor in this reaction. By using optimal amounts of Ca2+, phospholipid and Factor IXa, and an excess of Factor X, the rate of activation of Factor X is linearly related to the amount of Factor VIII. Factor Xa hydrolyses a chromogenic substrate thus liberating the chromophoric group. The color is then read photometrically at 405 nm. The generated Factor Xa and thus the intensity of color is proportional to the Factor VIII activity in the sample.

***In vitro* biopotency:** An AAV-specific *in vitro* biopotency assay was conducted using an AAV8 preparation evaluated using the antigen ELISA assay to determine the capsid particles per ml - cp/ml. The viral vector AAV8 comprising the gene of interest (GOI) Factor VIII (FVIII) infected a hepatic target cell line, which subsequently secreted functional and measurable FVIII protein into the cell culture medium. The factor VIII-activity of the cell culture supernatant was directly measured by a FVIII chromogenic assay. The measurement of an AAV8-FVIII sample is given as percentage relative to a reference material. The method allows a quantitative assessment of the biologic function of the AAV8-FVIII gene therapy vector. The same assay was used as described above for the *in vivo* biopotency.

As demonstrated in Figure 1, the use of qPCR methods resulted in greater variability as compared to the AAV-specific ELISA method. For example, the ITR-based qPCR method resulted in a coefficient of variance of ≤ 43%, while the coefficient of variance for the AAV8-specific ELISA was ≤ 10%. The ELISA method was about 20% to about 33% less variable than the qPCR method. This surprising difference allows the dose of an AAV fraction to be determined by the AAV-specific ELISA assay alone. This approach not only provides a more accurate way to dose an AAV fraction but it is more cost effective and time efficient.

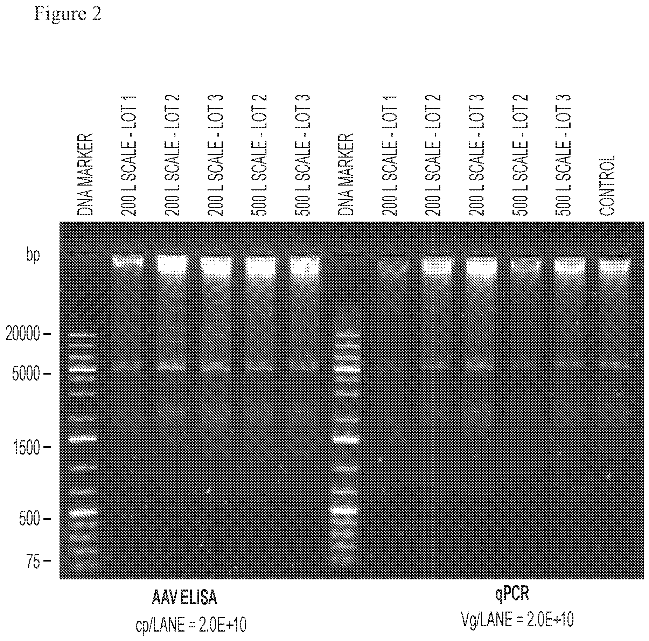

To test whether the ELISA method or qPCR method results in an accurate method of measuring the capsid content of a AAV preparation, AAV preparations were generated and evaluated using ELISA and separately qPCR. The results from these measurements were used to prepare a sample for loading into each lane (2.0 x 10¹⁰ capsids/lane). As shown in Figure 2, the samples evaluated with ELISA were consistently less variable than those evaluated with qPCR as the signal for the ELISA associated samples were more consistent as compared to those associated with the qPCR results, even though the preparations were from the same lots across both types of assays. Thus, dosing as aided with ELISA was more robust that the dosing associated with qPCR.

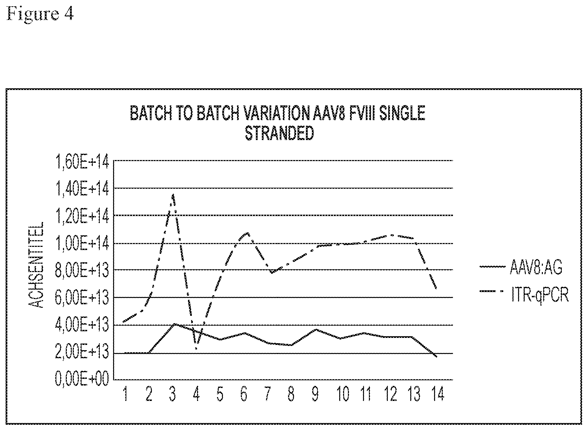

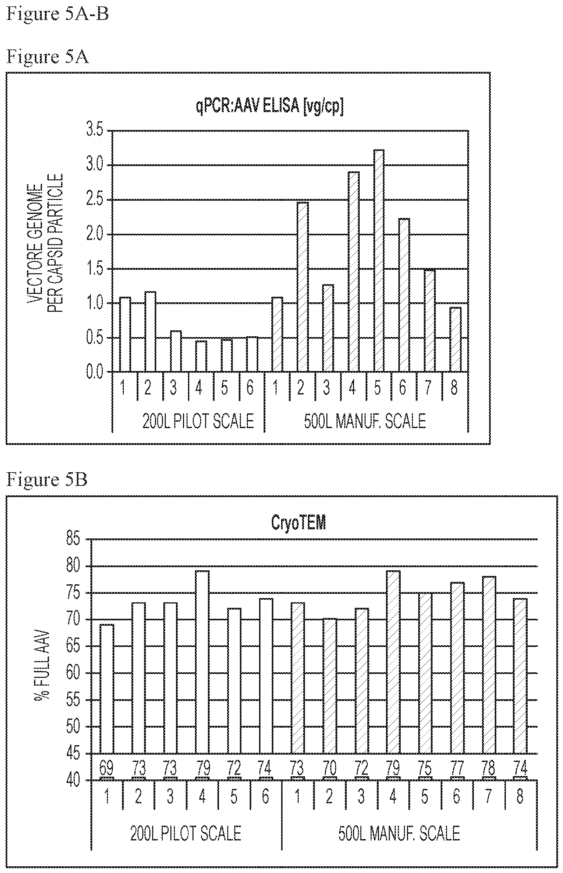

All results derived from release testing of preclinical and GMP bulk drug substance (BDS), and final drug product (FDP) lots by AAV8 capsid ELISA and ITR-qPCR are shown (Tables 1 to 4), and the ratios of vector genomes per total capsid particles were calculated (Table 5). For comparison, AAV8 ELISA values plotted against ITR-qPCR values are also shown (Figure 4). Figure 5 evaluates the ratio of AAV vector genomes (vg) per AAV8 antigen ELISA capsid particles (cp). Figure 5A shows the variability of the qPCR method on base of the ratio of vector genomes per AAV antigen. Figure 5B demonstrate the high consistency of the percentage of full AAV obtained from the manufacturing process according to the ultracentrifugation of PCT/US2017/059967 using the 50-55-60 method. The ultracentrifugation process provides a consistent full vs empty capsid profile from batch to batch. As such, the variability between assays should be consistent (e.g., Figure 5B). When qPCR, however, is used to calculate the dose, there is high variability between batches (e.g., Figure 5A).

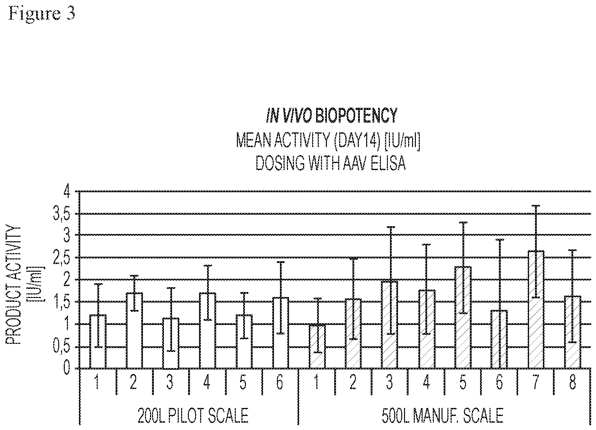

Next, mice were dosed in an *in vivo* biopotency assay using the ELISA method to measure the potency of the AAV preparation. Figure 3 verifies the use of the antigen ELISA method to determine the potency of an AAV preparation by using an *in vivo* biopotency assay using a mouse model. As indicated in Figure 3, the biological assay confirmed that there was biological activity in all lots and that the potency of the lots were comparable and consistent. As mentioned above, an *in vivo* biopotency assay evaluates both the ability of the AAV to transduce the cells and to produce the protein or gene of interest.

**Table 1: Bulk Drug Substance (BDS) lots: total AAV8 capsid particle titers measured by ELISA, and vector genome titers measured by ITR-qPCR, and their ratios. Means, standard deviations, coefficients of variations (CVs), and 3 standard deviation limits of the ratios are also shown. The ratio of ITR-qPCR/AAV8 ELISA provides the "specific activity" of the AAV preparation. The ratio or "specific activity" can provide information/number of full capsids but the variation is very high because it stems from both the qPCR and the ELISA. The variation comes mainly from the qPCR, which demonstrates why the AAV-ELISA or the AAV-ELISA/TEM method is superior over the qPCR method.**

| **BDSBatch#** | **Total AAV8 capsid particle ELISA [cp/ml]** | **Vector genomes by ITR-qPCR [vg/ml]** | **Ratio ITR-qPCR/AAV8 ELISA [vg/cp]** |
|---|---|---|---|
| 1 | 1.55E+13 | 3.53E+13 | 2.28 |
| 2 | 9.99E+12 | 2.45E+13 | 2.45 |
| 3 | 1.41E+13 | 1.80E+13 | 1.28 |
| 4 | 1.21E+13 | 1.39E+13 | 1.15 |
| 5 | 1.07E+13 | 9.20E+12 | 0.86 |
| 6 | 1.09E+13 | 1.28E+13 | 1.17 |
| | | **Mean (n=6)** | **1.53** |
| | | **Standard deviation SD** | **0.66** |
| | | **CV [%]** | **43** |

**Table 2: Good manufacturing practices (GMP) BDS lots: total AAV8 capsid particle titers measured by ELISA, and vector genome titers measured by ITR-qPCR, and their ratios. Means, standard deviations, coefficients of variations (CVs), and 3 standard deviation limits of the ratios are also shown.**

| **GMP BDS Lot#** | **Total AAV8 capsid particle ELISA [cp/ml]** | **Vector genomes by ITR-qPCR [vg/ml]** | **Ratio ITR-qPCR/AAV8 ELISA [vg/cp]** |
|---|---|---|---|
| 1 | 1.07E+13 | 1.15E+13 | 1.07 |
| 2 | 6.39E+13 | 7.31E+13 | 1.14 |
| 3 | 1.76E+13 | 4.05E+13 | 2.30 |
| 4 | 3.69E+13 | 4.73E+13 | 1.28 |
| 5 | 1.98E+13 | 5.76E+13 | 2.91 |
| 6 | 1.45E+13 | 4.69E+13 | 3.23 |
| 7 | 1.15E+13 | 2.56E+13 | 2.23 |
| | | **Mean (n=7)** | **2.02** |
| | | **Standard deviation SD** | **0.87** |
| | | **CV [%]** | **43** |

**Table 3: Final Drug Product (FDP) lots: total AAV8 capsid particle titers measured by ELISA, and vector genome titers measured by ITR-qPCR, and their ratios. Means, standard deviations, coefficients of variations (CVs), and 3 standard deviation limits of the ratios are also shown.**

| **FDP Lot#** | **Total AAV8 capsid particle ELISA [cp/ml]** | **Vector genomes by ITR-qPCR [vg/ml]** | **Ratio ITR-qPCR/AAV8 ELISA [vg/cp]** |
|---|---|---|---|
| 1 | 1.36E+13 | 2.83E+13 | 2.08 |
| 2 | 1.05E+13 | 3.09E+13 | 2.94 |
| 3 | 1.18E+13 | 1.93E+13 | 1.64 |
| 4 | 2.35E+12 | 2.45E+12 | 1.04 |
| 5 | 2.20E+12 | 2.22E+12 | 1.01 |
| 6 | 8.98E+13 | 1.74E+13 | 0.19 |
| | | **Mean (n=6)** | **1.48** |
| | | **Standard deviation SD** | **0.96** |
| | | **CV [%]** | **65** |

**Table 4: GMP FDP lots: total AAV8 capsid particle titers measured by ELISA, and vector genome titers measured by ITR-qPCR, and their ratios. Means, standard deviations, coefficients of variations (CVs), and 3 standard deviation limits of the ratios are also shown.**

| **GMP FDP Lot#** | **Total AAV8 capsid particle ELISA [cp/ml]** | **Vector genomes by ITR-qPCR [vg/ml]** | **Ratio ITR-qPCR/AAV8 ELISA [vg/cp]** |
|---|---|---|---|
| 1 | 6.38E+12 | 7.35E+12 | 1.15 |
| 2 | 8.64E+12 | 1.32E+13 | 1.53 |
| 3 | 2.40E+12 | 2.15E+12 | 0.90 |
| 4 | 1.08E+13 | 3.01E+13 | 2.79 |
| | | **Mean (n=4)** | **1.59** |
| | | **Standard deviation SD** | **0.84** |
| | | **CV [%]** | **53** |

**Table 5: Ratio of vector genomes per capsid particles for all BDS and FDP batches shown.**

| **All preclinical and GMP BDS Lots** | **Ratio ITR-qPCR/AAV8 ELISA [vg/cp]** |
|---|---|
| **Mean (n=23)** | **1.68** |
| **Standard deviation SD** | **0.82** |
| **CV [%]** | **49** |

**Table 6: In vitro and in vivo biopotency data of manufacturing batches of AAV8 containing vector DNA of B-Domain deleted FVIII. Dosing of AAV8 was based on AAV8 antigen ELISA, and the % full capsids was determined by CryoTEM. The batch to batch consistency in terms of %full capsids is demonstrated with CV 2.7%.**

| **Batch Nr.:** | **AAV8 Antigen** | **ITR - qPCR** | **in vitro BP** | **in vivo BP** | **CryoTEM Full** |
|---|---|---|---|---|---|
| | **cp/ml** | **vg/ml** | **BPU** | **IU/ml** | **%** |
| 17001 | 2.12E+13 | 4.27E+13 | 0.88 | 1.23±1.05 | 82 |
| 17002 | 2.13E+13 | 5.30E+13 | 0.86 | 2.09±1.15 | 78 |
| 17003 | 4.12E+13 | 1.35E+14 | 0.82 | 2.06±1.61 | 83 |
| 17004 | 3.56E+13 | 2.15E+13 | 1.04 | 2.1±0.65 | 84 |
| 17005 | 2.91E+13 | 8.06E+13 | 0.68 | 1.19±0.46 | 84 |
| 17006 | 3.36E+13 | 1.10E+14 | 1.01 | 1.74±0.81 | 85 |
| 17007 | 2.65E+13 | 7.74E+13 | 0.77 | 1.72±1.36 | 82 |
| 17008 | 2.46E+13 | 8.48E+13 | 0.84 | 1.66±0.48 | 82 |
| 17009 | 3.58E+13 | 9.73E+13 | 0.89 | 1.10±0.44 | 78 |
| 17010 | 2.99E+13 | 9.96E+13 | 1.23 | 2.27±1.12 | 81 |
| 17011 | 3.30E+13 | 9.96E+13 | 0.86 | 1.5±0.93 | 83 |
| 17012 | 3.11E+13 | 1.06E+14 | 0.96 | 2.03±1.03 | 83 |
| 17013 | 3.06E+13 | 1.03E+14 | 0.98 | 0.82±0.23 | 85 |
| 17014 | 1.71E+13 | 6.52E+13 | 1.98 | 1.28±0.90 | 80 |
| **Mean (n=14)** | | | | | 82.1 |
| **Standard deviation SD** | | | | | 2.2 |
| **CV [%]** | | | | | 2.7% |

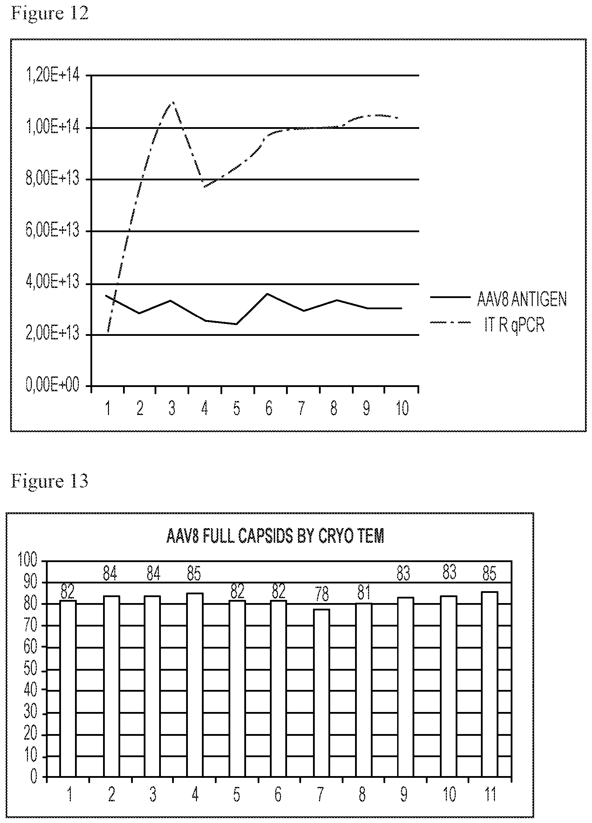

Figures 12 and 13 examine the ELISA, qPCR, and CryoTEM results from batches 17004-17014. Batches 17004-17013 (Table 6) are the same batch size, which are from 2 times 500L batches. These ten batches contain comparable amount of AAV8 capsids in the final product. Analysis of batches 17004-17013 is presented in Table 7.

**Table 7: In vitro and in vivo biopotency data of manufacturing batches of AAV8 containing vector DNA of B-Domain deleted FVIII for batches 17004-17013.**

| **Batches 17004-17013** | **AAV8 Antigen** | **ITR - qPCR** | **in vitro BP** | **in vivo BP** | **CryoTEM Full** |
|---|---|---|---|---|---|
| | **cp/ml** | **vg/ml** | **BPU** | **IU/ml** | **%** |
| MEDIAN | 3.09E+13 | 9.85E+13 | 0.93 | 1.69 | 83 |
| Standard deviation SD | 3.67E+12 | 2.58E+13 | 0.15 | 0.46 | 2.1 |

### EXAMPLE 4

This example validates CryoTEM as a tool to measure the amounts of full and empty capsids in an AAV preparation.

A combination of reliable analytical methods is required to measure quality attributes of gene therapy vector preparations based on recombinant AAV8 (rAAV8), to allow product release and accurate clinical dosing. CryoTEM was established to quantify full, vector genome-containing and empty vector particles in AAV8 gene therapy vector preparations, and to determine the significance of this parameter for potency using orthogonal methods.

CryoTEM was validated for full/empty quantification using rAAV8 vector preparations encoding human coagulation Factor IX (FIX) with contents of full particles of 40 - 80% generated by mixing vector preparations, or by selectively enriching vector fractions by ultracentrifugation. Validation resulted in intermediate and inter-assay precision below a relative standard deviation of 1%. Linearity of six independent experiments was achieved with each r² higher 0.996. Vector preparations derived from the same vector bulk showed a correlation of the full-to-empty ratio with the measured potency in a cell-based in vitro assay and in an in vivo model.

### Methods and materials

**CryoTEM:** Vector samples were preserved close to their native state by embedding them in amorphous, non-crystalline ice on an inert support by flash-freezing under cryogenic conditions. Image analysis based was done using an automated, objective analysis method using Vironova Analyzer Software (VAS) as described in Example 2.

**AAV8 ELISA:** Total AAV8 capsid particle titer was measured using a commercial kit (Progen) calibrated to the ATCC^{®} AAV8 reference standard material for capsid particle (cp) concentrations as described in Example 1.

***In vitro* biopotency:** The chromogenic activity of FIX in the supernatant of HepG2 cells infected with FIX-AAV8 was measured, given as arbitrary biopotency unit relative to a reference preparation, and normalized to capsid particles. In the *in-vitro* biopotency assay, the viral vector AAV8 infected a hepatic target cell line, which subsequently secretes functional, measurable encoded protein into the medium. In a first step HepG2 target cells are transduced infected by AAV8. During incubation, encoded protein was released into the supernatant. In a second step, the activity of the encoded protein secreted into the supernatant was directly measured by an activity assay (*in vitro* biopotency assay a enzymatic assay - ROX FIX (Rossix) was used). The measurement of an AAV8 sample is given as a percentage relative to a reference material. The method allows a quantitative assessment of the biologic function of the AAV8 gene therapy vector. The reference is the WHO Standard or a standard preparation calibrated against the WHO standard for human FIX.

***In vivo* biopotency:** Human FIX encoding AAV8 particles were infused i.v. in eight human FIX-knockout mice (Charles River Germany (CR0 in Sulzfeld, Germany)). The AAV8 preparation was evaluated by antigen ELISA to determine the capsid particles per ml - cp/ml. Samples were shipped to the testing facility (frozen, ≤-60°C), thawed and diluted with the formulation buffer to 4.95E+10 cp/ml. The animals received a single intravenous bolus injection of 2.47E+11cp/kg of the test or control samples, shortly after the samples were thawed. FIX-activity in plasma was monitored regularly via FIX-clotting activity measured by the one-stage APTT clotting assay against a human plasma standard calibrated against an international standard. A FIX containing sample was diluted to a range within a calibration curve in Imidazole buffer +1% albumin. 50µl of the diluted sample is added to 50µl FIX deficient plasma and contacted with a 50µl phospholipid suspension (Pathromtin SL, Siemens) after 240 seconds incubation the coagulation is started by adding 50µl of 25mM of CaCl2 solution, the time to generate a clotting formation is measured on a BCS XP(Siemens) or ACL 500 (Werfen)skid.

**qPCR:** A quantitative PCR assay based on Taqman^{®} chemistry was developed to measure vector genome copy concentrations of the transgene DNA. For the FIX vector DNA two FIX specific qPCR methods were used: one for the double stranded DNA AAV vector and another for the single stranded DNA AAV vector.

The vector genome titer (vg) per milliliter (ml) was determined using a TaqMan based qPCR with primers and a fluorescently labeled probe detecting a sequence within the FIX sequence and the polyadenylation signal. For detecting the FIX-specific sequence were treated with DNAse I and after a subsequent Proteinase K step the scAAV genome was released from the capsid. A finally restriction enzyme digest with is performed to resolve AAV ITR T-shape structures.

The plasmid used as reference Material was linearized with a single cutter restriction enzyme und further purified from an agarose Gel. UV A260 Absorbance of Gel extracted DNA was measured three times in a UV spectrophotometer and mean value of DNA concentration was calculated in µg per ml.

To determine the copy numbers per ml for the linearized standard plasmid the Molecular weight of ds DNA was calculated in gram per mol considering the exact mass for each individual nucleotide of the underlying sequence.

The vector genome titer in the test article (in vector genomes per mL) was calculated via plasmid standard curve fitting with linear regression.

### Results

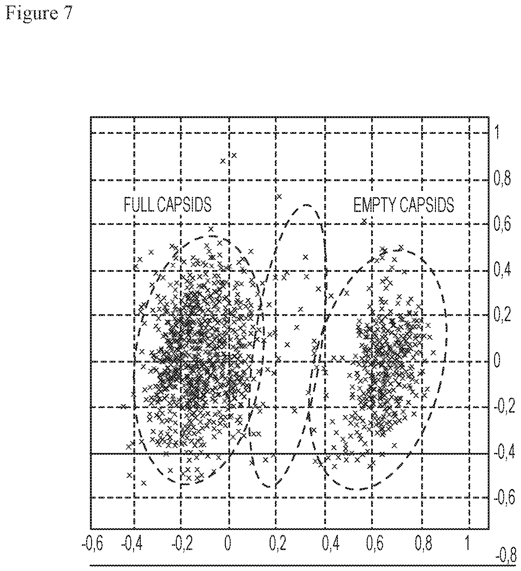

Figure 6 demonstrates that CryoTEM can be used to image full and empty capsids. Figure 7 is an example of a sample analysis. CryoTEM images acquired at 38k magnification were subjected to internal density analysis. Principal component analysis of each AAV particle's radial density profile revealed clusters of packaging levels: full (red; left), blue (empty; right), uncertain (green; middle); Dashed rings: 99% confidence intervals. See Table 8 for a summary of the findings.

**Table 8: Data obtained from 5 images analyzing a total of 1568 particles.**

| **Class** | **% (counts)** | **95% CI** |
|---|---|---|
| Empty | 20% (315) | 18-22% |
| Filled | 80% (1249) | 78-82% |
| Uncertain | 0% (4) | 0-1% |

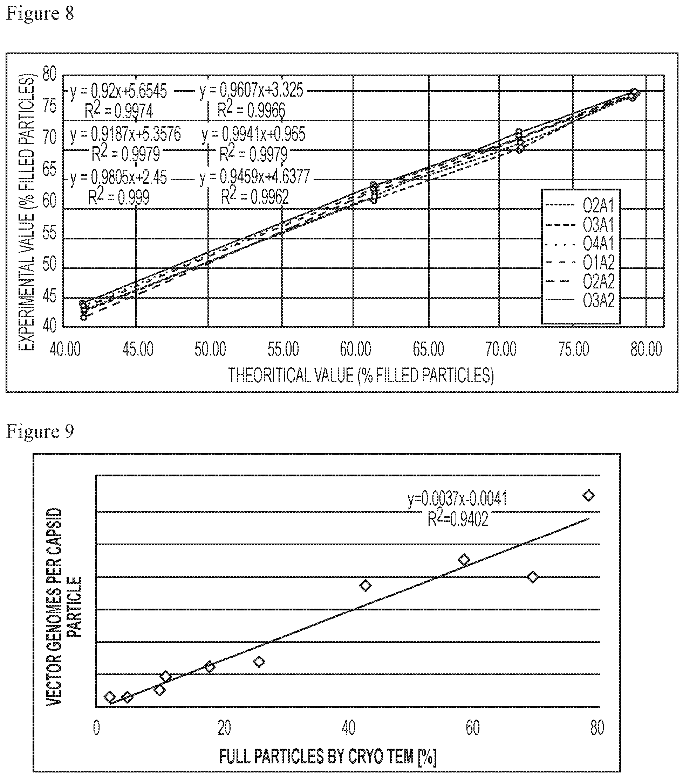

Figure 8 is a validation of CryoTEM according to ICH Q2(R1) (i.e., International Conference on Harmonization of Technical Requirements for Registration of Pharmaceuticals for Human Use) included specificity, repeatability, intermediate precision, linearity, dilutional accuracy, and robustness. Presented are the results for linearity from a set of six independent experiments. Figure 8 demonstrates the difference between the theoretical and the measured content of full AAV8 particles. rAAV8 preparations containing almost 80% full capsid, and another with mostly empty capsids, were mixed to obtain 4 samples with percentages of 40 to 80% full capsids. These preparations were measured on two grids each, wherein the x-axis represents the theoretical percentage of "full" capsids, and the y-axis represents the measured values by CryoTEM. One data point reflects a mean of two repeats. All demonstrated a good correlation of theoretical and experimental values with correlation coefficients above 0.996.

Figure 9 examines preparations with increasing degrees of full capsids that were purified from ultracentrifugation fractions (see the publication of international application PCT/US2017/059967). In particular, Figure 9 describes the increase of qPCR titer depending on the percentage of "full" capsids. The x-axis is the percentage of "full" capsids measured by CryoTEM, and the y-axis is the measured qPCR value. The higher the percentage of "full" capsids the higher is the qPCR titer. The ratio of qPCR per AAV8 Antigen in Figure 9 demonstrates how this ratio increases with the amount of "full" particles - the higher the percentage of full capsids the higher is the qPCR vs the AAV8 Antigen-ratio [ Y:Ratio vg/cp PER X:percentage of full capsids(CryoTEM)]. At higher percentages of full capsids, the variability of qPCR is higher, thus, making it less accurate. This especially important given that the product provided to the subject would be within the higher range of full capsids. Vector genomes per capsid particle titers were calculated from qPCR and AAV8 capsid particle ELISA results, and correlated to the CryoTEM data.

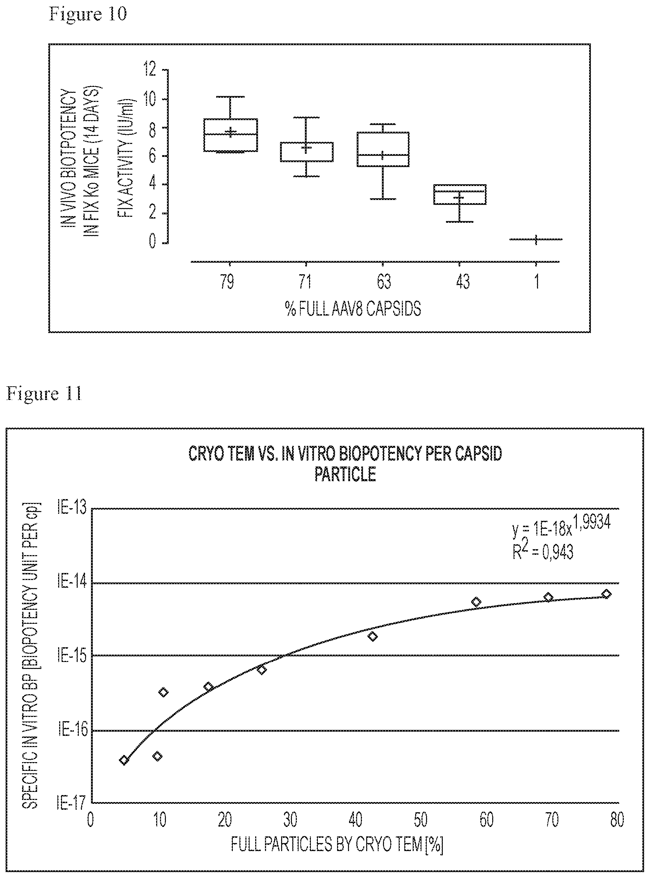

Figures 10 and 11 demonstrate the Influence of % full FIX-AAV8 capsid particles on the *in vivo* and the *in vitro* biopotency. In Figure 10, the same AAV vector preparation as used in Figure 8 was administered by i.v. to Factor IX knockout mice (n=8). Plasma samples were taken after 14 days and huFIX clotting activity was measured. For Figure 11, preparations with varying degrees of full capsids (same as in Figure 9) were prepared by isolation of ultracentrifugation fractions and subsequent purification. The biopotency was measured in a cell-based assay, related to cp, and plotted against the % of full particles. *In vitro* biopotency increases with higher degrees of full capsids.

**Table 9: Dosing for Figure 10 as determined by ELISA.**

| **Group** | **Test items** | **Mice** | **Doses** | ***In vivo* analyses Day 7, 14, 28** |
|---|---|---|---|---|
| A | S1= G9_VV_1602_BULK | 7 | 2.47E+11 cp/kg [ELISA titer] | retro-orbital puncture |
| | 78% full capsid | | | |
| B | S2= G9_VV_1602_meC_37_A | 7 | | |
| | 70% full/empty | | | FIX activity [clotting assay] FIX antigen [ELISA] |
| C | S3= G9_VV_1602_meC_37_B 60% full/empty | 7 | | |
| D | S4= G9_VV_1602_meC_37_C 40 % full/empty | 7 | | |
| E | S5= G9_VV_meC_37_BULK 0% empty capsid | 7 | | |

The % full capsids were measured using CryoTEM. In this case AAV8 containing double stranded DNA encoding FIX Padua was used. For the *in vivo* results of this experiment samples with different contents of full and empty particles were prepared by mixing a full capsid containing BDS product with an empty capsid BDS product as shown in Figure 10. The animals received a single intravenous bolus injection on day 0, and blood was collected on days 7, 14, and 28. The activity of FIX was analyzed in a FIX stage clotting assay (APTT-based factor assay is widely used for the measurement of factors VIII, IX, XI and XI). The sample was contacted with Factor IX deficient plasma and phospholipids, incubated, and the coagulation was started with a calcium chloride solution and the coagulation time is measured. The coagulation time have a clear mathematical relationship with the concentration of coagulation factors.

### Conclusions

CryoTEM is a valuable tool to quantify the potency of an AAV preparation, which will allow the release of a gene therapy vector preparation for therapeutic use. It can serve as an orthogonal method to measure amounts of full and empty capsid particles or vector genome titer for vector quantification. The CryoTEM has the advantage of allowing for the monitoring of gene therapy vector quality and potency.

CryoTEM is superior to other electron microscope based methods, as its sample preparation principle preserves to a great extent the native state of the sample. Moreover, there is no need to rely on stains.

## Claims

1. A method of quantifying the dose and/or potency of an adeno-associated virus (AAV) preparation, comprising quantifying a total number of AAV capsids in the AAV preparation via an AAV-specific ELISA assay and evaluating a percentage or ratio of full versus empty (full:empty) AAV capsids in the AAV preparation via cryogenic transmission electron microscopy (CryoTEM).

2. A method of measuring the concentration of full AAV capsids in an AAV preparation, comprising quantifying a total number of AAV capsids in the AAV preparation via an AAV-specific ELISA assay and evaluating a percentage of full versus empty AAV capsids in the AAV preparation via cryogenic transmission electron microscopy (CryoTEM).

3. A method for producing an AAV preparation, comprising:
(i) transfecting host cells in cell culture with at least one plasmid comprising a gene of interest;
(ii) collecting supernatant or cell suspension of the cell culture comprising AAV capsids to create an AAV fraction;
(iii) quantifying a total number of AAV capsids in the AAV fraction using an AAV-specific ELISA assay;
(iv) preparing an AAV preparation with a desired concentration based on the total number of AAV capsids; and
further comprising evaluating a percentage or ratio of full versus empty (full:empty) AAV capsids in the AAV preparation, wherein the evaluating step comprises determining the percentage or ratio of full: empty AAV capsids by CryoTEM, wherein optionally the evaluating step is conducted before or after the ELISA assay.

4. The method of claim 3, further comprising concentrating the AAV fraction; and/or further comprising removing at least a portion of empty capsids from the AAV fraction; and/or wherein the method further comprises lyophilizing the AAV preparation.

5. The method of any one of claims 1-4, wherein the quantification of full: empty AAV capsids is performed only once in the CryoTEM step to test that the batch has a consistent empty:full ratio as that of the previous batches.

6. The method of any one of claims 1-5, wherein the ELISA assay is a sandwich ELISA, direct ELISA, indirect ELISA, or competitive ELISA assay specific for an AAV antigen.

7. The method of any one of claims 1-6, wherein the CryoTEM step comprises:
(i) embedding the AAV preparation in a substrate in an inert support;
(ii) flash-freezing the embedded AAV preparation;
(iii) imaging the embedded AAV preparation using cryogenic transmission electron microscopy; and
(iv) quantitating the percentage of full AAV capsids versus empty AAV capsids in the AAV preparation,
wherein optionally said substrate is amorphous, non-crystalline ice,
wherein further optionally said AAV preparation is free from cellular debris.

8. The method of any one of claims 1-7, wherein the method does not include quantitative PCR.

9. The method of any one of claims 1-8, wherein the percentage of full AAV capsids is from about 40% to about 100% and/or wherein at least 60% of the AAV capsids are full AAV capsids.

10. The method of any one of claims 2-9, where in the concentration is between about 1×10¹⁰ capsids per ml (cp/ml) to about 1×10²⁰ cp/ml.

11. The method of any one of the preceding claims, wherein the AAV is AAV1, AAV2, AAV3, AAV4, AAV5, AAV6, AAV7, AAV8, AAV9, AAV10, or a chimeric AAV vector, wherein optionally said AAV is AAV8, or AAV9.

12. The method of any one of the preceding claims, wherein the AAV is a genetically engineered AAV, a chemically modified AAV, or both.

13. The method of any one of claims 6-12, wherein the AAV antigen is from an AAV1, AAV2, AAV3, AAV4, AAV5, AAV6, AAV7, AAV8, AAV9, AAV10, chimeric AAV, genetically engineered AAV, or chemically modified AAV.

## Patentansprüche

1. Verfahren zum Quantifizieren der Dosis und/oder Potenz eines Adeno-assoziierten Virus (AAV) Präparats, umfassend Quantifizieren einer Gesamtzahl von AAV Kapsiden in dem AAV Präparat mittels eines AAV-spezifischen ELISA Assays und Evaluieren eines Prozentsatzes oder Verhältnisses von vollen zu leeren (voll:leer) AAV Kapsiden in dem AAV Präparat mittels kryogener Transmissionselektronenmikroskopie (CryoTEM).

2. Verfahren zum Messen der Konzentration von vollen AAV Kapsiden in einem AAV Präparat, umfassend Quantifizieren einer Gesamtzahl von AAV Kapsiden in dem AAV Präparat mittels eines AAV spezifischen ELISA-Assay und Evaluieren eines Prozentsatzes von vollen zu leeren AAV Kapsiden in dem AAV Präparat mittels kryogener Transmissionselektronenmikroskopie (CryoTEM).

3. Verfahren zum Herstellen eines AAV Präparats, umfassend:
(i) Transfektieren von Wirtszellen in Zellkultur mit mindestens einem Plasmid, das ein Gen von Interesse umfasst;
(ii) Sammeln des Überstandes oder Zellsuspension der Zellkultur, die AAV Kapside umfassen, um eine AAV Fraktion zu kreieren;
(iii) Quantifizieren einer Gesamtzahl von AAV Kapsiden in der AAV Fraktion mittels eines AAV-spezifischen ELISA Assays;
(iv) Herstellen eines AAV Präparats mit einer gewünschten Konzentration auf der Grundlage der Gesamtzahl der AAV Kapside; und
ferner umfassend Evaluieren eines Prozentsatzes oder Verhältnisses von vollen zu leeren (voll:leer) AAV Kapsiden in dem AAV Präparat, wobei der Evaluierungsschritt Bestimmen des Prozentsatzes oder des Verhältnisses von vollen:leeren AAV Kapsiden durch CryoTEM umfasst, wobei optional der Evaluierungsschritt vor oder nach dem ELISA Assay durchgeführt wird.

4. Verfahren gemäß Anspruch 3, ferner umfassend Konzentrieren der AAV Fraktion; und/oder ferner umfassend Entfernen mindestens eines Teils der leeren Kapside aus der AAV Fraktion; und/oder wobei das Verfahren ferner Lyophilisieren des AAV Präparats umfasst.

5. Verfahren gemäß irgendeinem der Ansprüche 1-4, wobei das Quantifizieren der vollen:leeren AAV Kapsiden nur einmal im CryoTEM Schritt durchgeführt wird, um zu prüfen, ob die Charge ein konsistentes voll:leer Verhältnis wie das der vorherigen Chargen aufweist.

6. Verfahren gemäß irgendeinem der Ansprüche 1-5, wobei der ELISA Assay ein Sandwich ELISA, direkter ELISA, direkter ELISA, indirekter ELISA oder kompetitiver ELISA Assay ist, der für ein AAV Antigen spezifisch ist.

7. Verfahren gemäß irgendeinem der Ansprüche 1-6, wobei der CryoTEM-Schritt umfasst:
(i) Einbetten des AAV Präparats in ein Substrat auf einem inerten Träger;
(ii) Schnellgefrieren des eingebetteten AAV Präparats;
(iii) Abbilden des eingebetteten AAV Präparats unter Verwendung der kryogenen Transmissionselektronenmikroskopie; und
(iv) Quantifizieren des prozentualen Anteils an vollen AAV Kapsiden gegenüber leeren AAV Kapsiden in dem AAV Präparat,
wobei optional das Substrat amorphes, nicht kristallines Eis ist,
wobei ferner optional das AAV Präparat frei von Zelltrümmern ist.

8. Verfahren gemäß irgendeinem der Ansprüche 1-7, wobei das Verfahren keine quantitative PCR umfasst.

9. Verfahren gemäß irgendeinem der Ansprüche 1-8, wobei der Prozentsatz der vollen AAV Kapside von etwa 40 % bis etwa 100 % ist und/oder wobei mindestens 60 % der AAV Kapside volle AAV Kapside sind.

10. Verfahren gemäß irgendeinem der Ansprüche 2-9, wobei die Konzentration zwischen etwa 1×10¹⁰ Kapsiden pro ml (cp/ml) bis etwa 1×10²⁰ cp/ml ist.

11. Verfahren gemäß irgendeinem der vorhergehenden Ansprüche, wobei das AAV AAV1, AAV2, AAV3, AAV4, AAV5, AAV6, AAV7, AAV8, AAV9, AAV10, oder ein chimärer AAV Vektor ist, wobei optional das AAV AAV8 oder AAV9 ist.

12. Verfahren gemäß irgendeinem der vorhergehenden Ansprüche, wobei das AAV ein gentechnisch verändertes AAV, ein chemisch modifiziertes AAV oder beides ist.

13. Verfahren gemäß irgendeinem der Ansprüche 6-12, wobei das AAV Antigen aus einem AAV1, AAV2, AAV3, AAV4, AAV5, AAV6, AAV7, AAV8, AAV9, AAV10, chimärem AAV, gentechnisch verändertes AAV oder chemisch modifiziertes AAV ist.

## Revendications

1. Procédé destiné à quantifier la dose et/ou l'activité thérapeutique d'une préparation de virus adéno-associés (AAV), comprenant une quantification d'un nombre total de capsides d'AAV dans la préparation d'AAV par le biais d'un test ELISA spécifique AAV et une évaluation d'un pourcentage ou ratio de capsides d'AAV pleines par rapport à des vides (pleines:vides) dans la préparation d'AAV par le biais d'une cryomicroscopie électronique à transmission (CryoTEM).

2. Procédé destiné à mesurer la concentration de capsides d'AAV pleines dans une préparation d'AAV, comprenant une quantification d'un nombre total de capsides d'AAV dans la préparation d'AAV par le biais d'un test ELISA spécifique AAV et une évaluation d'un pourcentage de capsides d'AAV pleines par rapport à des vides dans la préparation d'AAV par le biais d'une cryomicroscopie électronique à transmission (CryoTEM).

3. Procédé pour produire une préparation d'AAV, comprenant :
(i) une transfection de cellules hôtes dans une culture cellulaire avec au moins un plasmide comprenant un gène d'intérêt ;
(ii) une collecte de surnageant ou de suspension cellulaire de la culture cellulaire comprenant des capsides d'AAV pour créer une fraction d'AAV ;
(iii) une quantification d'un nombre total de capsides d'AAV dans la fraction d'AAV en utilisant un test ELISA spécifique AAV ;
(iv) une préparation d'une préparation d'AAV avec une concentration souhaitée basée sur le nombre total de capsides d'AAV ; et
comprenant en outre une évaluation d'un pourcentage ou ratio de capsides d'AAV pleines par rapport à des vides (pleines:vides) dans la préparation d'AAV, dans lequel l'étape d'évaluation comprend une détermination du pourcentage ou ratio de capsides d'AAV pleines:vides par CryoTEM, dans lequel facultativement l'étape d'évaluation est réalisée avant ou après le test ELISA.

4. Procédé selon la revendication 3, comprenant en outre une concentration de la fraction d'AAV ; et/ou comprenant en outre une élimination d'au moins une partie de capsides vides de la fraction d'AAV ; et/ou dans lequel le procédé comprend en outre une lyophilisation de la préparation d'AAV.

5. Procédé selon l'une quelconque des revendications 1 à 4, dans lequel la quantification de capsides d'AAV pleines:vides est réalisée seulement une fois dans l'étape CryoTEM pour tester que le lot a un ratio vides:pleines cohérent avec celui des lots précédents.

6. Procédé selon l'une quelconque des revendications 1 à 5, dans lequel le test ELISA est un test ELISA sandwich, un test ELISA direct, un test ELISA indirect, ou un test ELISA compétitif spécifique de l'antigène AAV.

7. Procédé selon l'une quelconque des revendications 1 à 6, dans lequel l'étape CryoTEM comprend :
(i) une incorporation de la préparation d'AAV dans un substrat dans un support inerte ;
(ii) une congélation ultra-rapide de la préparation d'AAV incorporée ;
(iii) une imagerie de la préparation d'AAV incorporée en utilisant une cryomicroscopie électronique à transmission ; et
(iv) une quantification du pourcentage de capsides d'AAV pleines par rapport aux capsides d'AAV vides dans la préparation d'AAV,
dans lequel facultativement ledit substrat est de la glace amorphe non cristalline,
dans lequel en outre facultativement ladite préparation d'AAV est exempte de débris cellulaires.

8. Procédé selon l'une quelconque des revendications 1 à 7, dans lequel le procédé n'inclut pas de PCR quantitative.

9. Procédé selon l'une quelconque des revendications 1 à 8, dans lequel le pourcentage de capsides d'AAV pleines est entre environ 40 % et environ 100 % et/ou dans lequel au moins 60 % des capsides d'AAV sont des capsides d'AAV pleines.

10. Procédé selon l'une quelconque des revendications 2 à 9, où la concentration est entre environ 1×10¹⁰ capsides par ml (cp/ml) et environ 1×10²⁰ cp/ml.

11. Procédé selon l'une quelconque des revendications précédentes, dans lequel l'AAV est AAV1, AAV2, AAV3, AAV4, AAV5, AAV6, AAV7, AAV8, AAV9, AAV10, ou un vecteur d'AAV chimère, dans lequel facultativement ledit AAV est AAV8, ou AAV9.

12. Procédé selon l'une quelconque des revendications précédentes, dans lequel l'AAV est un AAV génétiquement conçu, un AAV chimiquement modifié, ou les deux.

13. Procédé selon l'une quelconque des revendications 6 à 12, dans lequel l'antigène AAV provient d'un AAV1, AAV2, AAV3, AAV4, AAV5, AAV6, AAV7, AAV8, AAV9, AAV10, AAV chimère, AAV génétiquement conçu, ou AAV chimiquement modifié.
